(19) Europäisches Patentamt
European Patent Office
Office européen des brevets

(11) Publication number: **0 269 298 B1**

# (12) EUROPEAN PATENT SPECIFICATION

(45) Date of publication of patent specification: **19.10.94**

(51) Int. Cl.$^5$: **C07D 501/46**, C07D 501/36, C07D 501/34, C07D 501/20, A61K 31/545, C07D 501/56

(21) Application number: **87309852.9**

(22) Date of filing: **06.11.87**

The file contains technical information submitted after the application was filed and not included in this specification

(54) **Cephalosporins, process for their preparation and pharmaceutical compositions.**

(30) Priority: **21.11.86 EP 86402592**

(43) Date of publication of application:
**01.06.88 Bulletin 88/22**

(45) Publication of the grant of the patent:
**19.10.94 Bulletin 94/42**

(84) Designated Contracting States:
**AT BE CH DE ES FR GB GR IT LI LU NL SE**

(56) References cited:
**EP-A- 0 238 060**
**EP-A- 0 241 901**
**DE-A- 3 137 854**
**FR-A- 2 552 088**

**CHEMICAL ABSTRACTS, vol. 107, no. 23, 7th December 1987, page 563, abstract no. 217359m, Columbus, Ohio, US; & JP-A-62 51 688**

(73) Proprietor: **ICI PHARMA**
**Immeuble "Le Galien"**
**B.P. 127**
**1 Rue des Chauffours**
**F-95022 Cergy Cédex (FR)**

(72) Inventor: **Bertrandie, Alain Michel**
**16 rue Jacques Brel**
**F-51350 Cormontreuil (FR)**
Inventor: **Bird, Thomas Geoffrey Colerick**
**7 Rue Pierre Boileau**
**F-51420 Witry-Les-Reims (FR)**
Inventor: **Jung, Frederic Henry**
**Rue du Moulin Cliquot**
**Taissy**
**F-51500 Rilly La Montagne (FR)**

(74) Representative: **Denerley, Paul Millington, Dr. et al**
**ICI Group Patents Services Dept.**
**PO Box 6**
**Shire Park**
**Bessemer Road**
**Welwyn Garden City Herts, AL7 1HD (GB)**

EP 0 269 298 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to cephalosporins and in particular to such compounds comprising a catechol or related group. This invention further relates to processes for their preparation, to intermediates in their preparation, to their use as therapeutic agents and to pharmaceutical compositions containing them. The compounds of this invention are antibiotics and can be used in the treatment of any disease that is conventionally treated with antibiotics for example in the treatment of bacterial infection in mammals including humans. The compounds of this invention also have non-therapeutic uses as they can be used in conventional manner in industry for example they can be used as disinfectants and food preservatives. The compounds of this invention, however, are primarily of therapeutic interest as they show a desirable profile of activity and duration in their antibacterial effect.

Investigation into new cephalosporin derivatives has been intense over the past 25 years with many thousands of patents and scientific papers having been published. A particular problem associated with the commercially available cephaolsporins is the lack of potency against strains of Pseudomonas. The present invention provides cephalosporin derivatives having novel 3-position substituents, which derivatives possess good antibacterial activity and in particular against strains of Pseudomonas.

USP4278793 discloses cephalosporins having a 3-position substituent of the formula : $-CH_2Y$ wherein Y can be the residue of a nucleophilic compound, preferably a sulphur, nitrogen or oxygen nucleophilic compound. The emphasis therein is directed to heterocyclicthiomethyl compounds; however optionally substituted arylthiomethyl compounds are specifically mentioned. GB1496757 discloses cephalosporins having a 3-position substituent of the formula: $-CH_2Y$ wherein Y can be the residue of a nucleophilic compound, this specification includes a discussion of nitrogen, carbon, oxygen and sulphur nucleophiles. Many possible values of Y are mentioned, including arylthio, aryl lower alkylthio, aryloxy and aryl lower alkoxy. GB 2148282 discloses cephalosporin compounds having a 3-position substituent of the formula: $-CH_2R^7$ wherein inter alia $R^7$ is optionally substituted aryl for example phenyl. DE 3137854 discloses cephalosporins having a 3-position substituent of the formula $-CH_2R^2$ wherein $R^2$ can be aryl. A specific example in this specification has the 3-position substituent 4-hydroxybenzyl. Cephalosporins with the 3-position substituent $-CH_2R^7$ are disclosed in FR 2552088, wherein $R^7$ can be aryl. The thrust of both of these applications is towards triazolylmethyl and tetrazolylmethyl 3-position substituents. The above specifications are typical of many specifications that describe cephalosporins having nucleophilic moities linked via a methylene group to the 3-position of a cephalosporin. However, although there has been intense research over a long period of time, there has been no teaching or suggestion of the compounds of the present invention. These contain specific ring systems that are characterised by having hydroxy groups or related substituents ortho to one another. These hitherto undisclosed ring systems give rise to particularly good activity against strains of Pseudomonas. Mention should be made of Japanese Kokai 62-051688, published after the priority date of the present invention, which discloses 7-(2-(2-aminothiazol-4-yl)-2-(syn)-methoxy-iminoacetamido-3-(3,4-dihydroxyphenyl)methyl-ceph-3-em-4-carboxylic acid.

Accordingly the present invention provides a cephalosporin compound of the formula (IV):

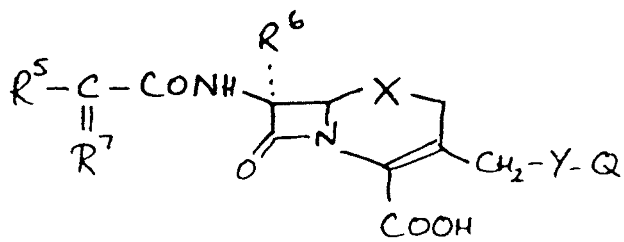

(IV)

and salts and esters thereof wherein:

X is sulphur, oxygen, methylene or sulphinyl;

$R^6$ is hydrogen, methoxy or formamido; and $R^5$ and $R^7$ are groups known for such positions in the cephalosporin art.

Preferably X is sulphur.

Preferably $R^6$ is hydrogen.

$R^5$ is for example 2-aminothiazol-4-yl or 2-aminooxazol-4-yl each optionally substitued in the 5-position by fluorine, chlorine or bromine, or $R^5$ is 5-aminoisothiazol-3-yl, 5-amino-1,2,4-thiadiazol-3-yl, 3-aminopyrazol-4-yl, 2-aminopyrimidin-5-yl, 2-aminopyrid-6-yl, 4-aminopyrimidin-2-yl, 2-amino-1,3,4-thiadiazol-5-yl or 5-amino-1-methyl-1,2,4-triazol-3-yl;

$R^7$ is for example of the formula =N.O.$R^8$ (having the syn configuration about the double bond) wherein $R^8$ is hydrogen, (1-6C)alkyl, (3-8C)cycloalkyl, (1-3C)alkyl(3-6C)cycloalkyl, (3-6C)cycloalkyl(1-3C)-alkyl, (3-6C)alkenyl, optionally substituted by carboxy, (5-8C)cycloalkenyl, (3-6C)alkynyl, (2-5C)-alkylcarbamoyl, phenylcarbamoyl, benzylcarbamoyl, (1-4C)alkylcarbamoyl(1-4C)alkyl, di(1-4C)-alkylcarbamoyl(1-4C)alkyl, (1-4C)haloalkylcarbamoyl(1-4C)alkyl, (1-3C)haloalkyl, (2-6C)hydroxyalkyl, (1-4C)-alkoxy(2-4C)alkyl, (1-4C)alkylthio(2-4C)alkyl, (1-4C)alkanesulphinyl(1-4C)alkyl, (1-4C)alkanesulphonyl(1-4C)-alkyl), (2-6C)aminoalkyl, (1-4C)alkylamino(1-6C)alkyl, (2-8C)dialkylamino(2-6C)alkyl, (1-5C)cyanoalkyl, 3-amino-3-carboxypropyl, 2-(amidonothio)ethyl, 2-(N-aminoamidinothio)ethyl, tetrahydropyran-2-yl, thietan-3-yl, 2-oxopyrrolidinyl, or 2-oxotetrahydrofuranyl, or $R^8$ is of the formula V:-

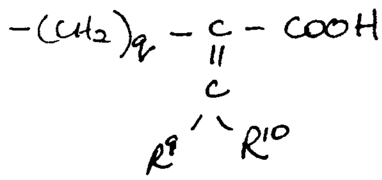

(V)

wherein q is one or two and $R^9$ and $R^{10}$ are indepently hydrogen or $C_{1-4}$alkyl; or $R^8$ is of the formula VI:-

$-CR^{11}R^{12}-(CH_2)_r-COR^{13}$ (VI)

wherein r is 0-3, $R^{11}$ is hydrogen, (1-3C)alkyl or methylthio, $R^{12}$ is hydrogen (1-3C)alkyl, (3-7C)cycloalkyl, cyano, carboxy, (2-5C)carboxyalkyl or methanesulphonylamino, or $R^{11}$ and $R^{12}$ are joined to form, together with the carbon to which they are attached, a (3-7C)carbocyclic ring, and $R^{13}$ is hydroxy, amino, (1-4C)-alkoxy, (1-4C)alkylamino or of the formula NHOR$^{14}$ in which $R^{14}$ is hydrogen or (1-4C)alkyl;

or $R^7$ may be of the formula =CH.$R^{15}$ wherein $R^{15}$ is hydrogen, halogen, (1-6C)alkyl, (3-7C)cycloalkyl, (2-6C)alkenyl, (3-7C)cycloalkenyl, phenyl or benzyl;

Q is:

(i) a benzene ring (optionally fused to a further benzene ring so forming a naphthyl group or optionally fused to a 5 or 6 membered heterocyclic aromatic group containing 1, 2 or 3 heteroatoms selected from nitrogen, oxygen and sulphur), said benzene ring being substituted by groups $R^1$ and $R^2$ which are ortho with respect to one another,

wherein $R^1$ is hydroxy and $R^2$ is hydroxy;

(ii) a group of the formula (II):

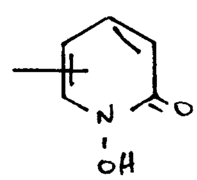

or;

(iii) a group of the formula (III):

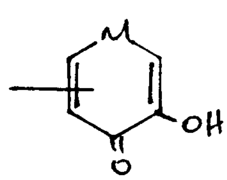

wherein M is oxygen or a group NR$^3$

wherein $R^3$ is hydrogen or $C_{1-4}$ alkyl:

ring Q (or, in the case wherein ring Q is a benzene ring fused to another benzene ring, either benzene ring) is optionally urther substituted by

$C_{1-4}$ alkyl, halo, hydroxy, hydroxy $C_{1-4}$ alkyl, cyano, trifluoromethyl, nitro, amino, $C_{1-4}$ alkylamino,

di-$C_{1-4}$ alkylamino, amino $C_{1-4}$ alkyl, $C_{1-4}$ alkylamino $C_{1-4}$ alkyl, di-$C_{1-4}$ alkylamino $C_{1-4}$ alkyl, alkyl, $C_{1-4}$ alkanoyl, $C_{1-4}$ alkoxy, $C_{1-4}$ alkylthio, $C_{1-4}$ alkanoyloxy, carbamoyl, $C_{1-4}$ alkylcarbamoyl, di-$C_{1-4}$ alkyl carbamoyl, carboxy, carboxy $C_{1-4}$ alkyl, sulpho, sulpho $C_{1-4}$ alkyl, $C_{1-4}$ alkanesulphonamido, $C_{1-4}$ alkoxycarbonyl, $C_{1-4}$ alkanoylamino, nitroso, thioureido, amidino, ammonium, mono-, di- or tri- $C_{1-4}$ alkylammonium or pyridinium, or a 5-membered heterocyclic ring containing 1 to 4 heteroatoms selected from oxygen, nitrogen and sulphur which is optionally substituted by 1, 2 or 3 $C_{1-4}$ alkyl or $C_{1-4}$ alkoxy groups,

Y, which links into the benzene ring or the ring of formula (II) or (III), is a covalent bond or is a group -V-Y'- wherein V is oxygen, sulphur or a group $-NR^4-$ and -Y'- is a covalent bond or $C_{1-4}$ alkenylene, wherein $R^4$ is hydrogen, $C_{1-4}$ alkyl optionally substituted by any one of halo, hydroxy, $C_{1-4}$ alkoxy, carboxy, amino, cyano, $C_{1-4}$ alkanoylamino or phenyl or $R^4$ is $C_{2-6}$ alkenyl $C_{1-4}$ alkanesulphonyl or $C_{1-4}$ alkanoyl.

In one aspect V is oxygen, sulphur or a group $-NR^4-$ and Y' is a covalent bond. In a preferred aspect Y is a covalent bond. In another preferred aspect V is oxygen, sulphur or a group $-NR^4-$ and Y' is a $C_{1-4}$alkylene group, in particular Y' is methylene.

A particularly preferred linking group Y is $-NR^4-CH_2-$.

Suitable examples of $R^4$ include hydrogen, methyl, ethyl, n-propyl, isopropyl, 2-fluoroethyl, 2-chloroethyl, 2-hydroxymethyl, 2-methoxyethyl, carboxymethyl, 2-aminoethyl, 2-cyanoethyl, 2-formamidoethyl, allyl, acetyl, propionyl, methanesulphonyl, fufuryl and benzyl. Favourably $R^4$ is hydrogen, methyl, ethyl, acetyl or methanesulphonyl.

In one aspect ring Q is a benzene ring substituted by groups $R^1$ and $R^2$ as hereinbefore defined. $R^1$ is hydroxy or an in vivo hydrolysable ester thereof. In vivo hydrolysable esters are those pharmaceutically acceptable esters that hydrolyse in the human or animal body to produce the parent hydroxy compound. Such esters can be identified by administering, e.g. intravenously to a test animal, the compound under test and subsequently examining the test animal's body fluids. Suitable in vivo hydrolysable esters include $C_{1-6}$ alkanoyloxy for example acetoxy, propionyloxy, pivaloyloxy, $C_{1-4}$ alkoxycarbonyloxy for example ethoxycarbonyloxy, phenylacetoxy and phthalidyl.

In one aspect Q is a ring of the formula (III). Suitably M is oxygen thus forming a pyranone ring. Suitably also M is -NH- in which case the linking group may be attached to the pyranone ring via the ring nitrogen atom or via a ring carbon atom, in the latter case allowing a greater degree of tautomerism into the hydroxypyridine tautomer. In a further aspect M is $-NR^3$ wherein $R^3$ is $C_{1-4}$ alkyl in which case the linking group is Y is attached to the pyranone ring via a ring carbon atom.

In a preferred aspect Q is a benzene ring optionally fused to another benzene ring so forming a naphthyl group. As stated hereinbefore either benzene group may be substituted by $R^1$ and $R^2$ and by other optional substituents. Particular optional substituents are $C_{1-4}$ alkyl for example methyl, ethyl or isopropyl, halo for example chloro, bromo or fluoro, hydroxy, hydroxy $C_{1-4}$ alkyl for example hydroxymethyl, amino, nitro, $C_{1-4}$ alkoxy for example methoxy or ethoxy, carboxy $C_{1-4}$ alkyl for example carboxymethyl, $C_{1-4}$ alkanoylamino for example acetamido, trifluoromethyl, carboxy, carbamoyl, cyano, sulpho, $C_{1-4}$ alkanesulphonamido for example methanesulphonamido, $C_{1-4}$ alkanoyl for example acetyl, $C_{1-4}$ alkanoyloxy for example acetoxy or propionoxy and $C_{1-4}$ alkoxycarbonyl for example methoxycarbonyl. Of these, favoured substituents are sulpho, carboxymethyl, methyl, ethyl, methoxy, bromo, chloro, fluoro and nitro.

The skilled man will realise that when Q is a benzene ring up to 3 optional substituents are possible; when a naphthyl ring is formed more substituents are possible and up to 2 or 3 substituents are possible with the rings of formulae (II) and (III). In general, we prefer up to 2 optional substituents, which may be the same or different.

Particular meanings for $R^8$ are hydrogen, methyl, ethyl, isopropyl, t-butyl, cyclopropyl, cyclobutyl, cyclopentyl, cyclohexyl, methylcyclopropyl, methylcyclobutyl, methylcyclopentyl, methylcyclohexyl, cyclopropylmethyl, cyclobutylmethyl, cyclopentylmethyl, allyl, cyclopentenyl, cyclohexenyl, propargyl, methylcarbamoyl, ethylcarbamoyl, phenylcarbamoyl, benzylcarbamoyl, 2-chloroethyl, 2-fluoroethyl, 2-bromoethyl, 2-hydroxyethyl, 3-hydroxypropyl, 2-methoxyethyl, 2-ethoxyethyl, 2-methylthio-ethyl, 2-methanesulphinylethyl, 2-methanesulphonyl-ethyl, 2-aminoethyl, 3-aminopropyl, 2-methylamino ethyl, 2-dimethylaminoethyl, cyanomethyl, 2-cyanoethyl, azidomethyl, 2-azidoethyl, ureidomethyl, 3-amino-3-carboxypropyl, 2-(amidino)ethyl, 2-(N-aminoamidino)-ethyl, tetrahydropyran-2-yl, thietan-3-yl, 2-oxopyrrolidinyl and 2-oxotetrahydrofuran-3-yl,

or, when $R^8$ is of the formula V in which q is 1 or 2, a particular meaning for $R^8$ is when $R^9$ and $R^{10}$ are hydrogen or methyl,

or, when $R^8$ is of the formula VI, a particular meaning for $R^8$ is when $r=0$ and $R^{11}$ is hydrogen, methyl

or methylthio, $R^{12}$ is hydrogen, methyl, cyclopropyl, cyclobutyl, cyclopentyl, cyclohexyl, cyano, carboxy, carboxymethyl, 2-carboxyethyl or methanesulphonylamino, or when $R^{11}$ and $R^{12}$ are joined to form, together with the carbon to which they are attached, a cyclopropane, cyclobutane, cyclopentane, cyclohexane or cycloheptane ring and $R^{13}$ is hydroxy, amino, methoxy, ethoxy, methylamino, ethylamino, or of the formula $NHOR^{14}$ in which $R^{14}$ is hydrogen, methyl or ethyl.

Preferably $R^8$ is $C_{1-6}$ alkyl for example methyl or ethyl, 1-carboxycyclobutyl, 1-carboxycyclopentyl, or 2-carboxyprop-2-yl. In particular $R^8$ is 2-carboxyprop-2-yl.

Particular meanings for $R^{15}$ are hydrogen, methyl, ethyl or chlorine.

Preferred compounds of the present invention include: 3-(3,4-dihydroxybenzoylaminomethyl)-7-[2-(2-aminothiazol-4-yl)-2-((Z)-1-carboxy-1-methylethoxyimino)acetamido]ceph-3-em-4-carboxylic acid; and 3-(3,4-dihydroxybenzyl)-7-[2-(2-aminothiazol-4-yl)-2-((Z)-1-carboxy-1-methylethoxyimino)acetamido]ceph-3-em-4-carboxylic acid.

The cephalosporin derivatives referred to herein are generally named in accordance with the "cephem" nomenclature and numbering system proposed in J.A.C.S. 1962, 84, 3400.

It will be realised, of course, that the present invention covers all isomeric and tautomeric forms of the aforementioned compounds. For example the rings of the formula (III) may be in pyranone or hydroxypyridine form.

As stated hereinbefore the compounds of this invention ar primarily intended for use in therapy. Therefore in a preferred aspect the present invention provides a cephalosporin compound having a 3-position substituent of the formula I or a pharmaceutically acceptable salt or ester thereof. Suitable salts include acid addition salts such as hydrochloride, hydrobromide, citrate, maleate and salts formed with phosphoric and sulphuric acid. In another aspect suitable salts are base salts such as an alkali metal salt for example sodium or potassium, an alkaline earth metal salt for example calcium or magnesium, or organic amine salt for example triethylamine, morpholine, N-methylpiperidine, N-ethylpiperidine, procaine, dibenzylamine, or N,N-dibenzylethylamine.

In order to use a compound of the present invention or a pharmaceutically acceptable salt or ester thereof for the therapeutic treatment of mammals including humans, in particular in treating infection, it is normally formulated in accordance with standard pharmaceutical practice as a pharmaceutical composition.

Therefore in another aspect the present invention provides a pharmaceutical composition which comprises a cephalosporin compound having a 3-position substituent of the formula I or a pharmaceutically acceptable salt or ester thereof and a pharmaceutically acceptable carrier.

The pharmaceutical compositions of this invention may be administered in standard manner for the disease condition that it is desired to treat, for example by oral, rectal or parenteral administration. For these purposes it may be formulated by means known to the art into the form of, for example, tablets, capsules, aqueous or oily solutions or suspensions, emulsions, dispersible powders, suppositories and sterile injectable aqueous or oily solutions or suspensions.

In addition to the pharmaceutically acceptable cephalosporin derivative of the present invention the pharmaceutical composition of the invention may also contain, or be co-administered with, one or more known drugs selected from other clinically useful antibacterial agents (for example other beta-lactams or aminoglycosides), inhibitors of beta-lactamase (for example clavulanic acid), renal tubular blocking agents (e.g. probenicid) and inhibitors of metabolising enzymes (for example inhibitors of peptidases, for example Z-2-acylamino-3-substituted propenoates).

A preferred pharmaceutical composition of the invention is one suitable for intravenous, subcutaneous or intramuscular injection, for example a sterile injectable containing between 1 and 50% w/w of the cephalosporin derivative, or one suitable for oral administration in unit dosage form, for example a tablet or capsule which contains between 100 mg. and 1 g. of the cephalosporin derivative.

The pharmaceutical compositions of the invention will normally be administered to man in order to combat infections caused by bacteria, in the same general manner as that employed for cephalothin, cefoxitin, cephradine, ceftazidime and other known clinically used cephalosporin derivatives, due allowance being made in terms of dose levels for the potency of the cephalosporin derivative of the present invention relative to the known clinically used cephalosporins. Thus each patient will receive a daily intravenous, subcutaneous or intramuscular dose of 0.05 to 30 g., and preferably 0.1 to 10 g., of the cephalosporin derivative, the composition being administered 1 to 4 times per day, preferably 1 or 2 times a day. The intravenous, subcutaneous and intramuscular dose may be given by means of a bolus injection. Alternatively the intravenous dose may be given by continuous infusion over a period of time. Alternatively each patient will receive a daily oral dose which is approximately equivalent to the daily parenteral dose. Thus a preferred daily oral dose is 0.5 to 10 g. of the cephalosporin derivative, the composition being administered 1 to 4 times per day.

In a further aspect the present invention provides a process for preparing a cephalosporin compound of the formula (IV), which process comprises:

(a) reacting a cephalosporin compound of the formula:

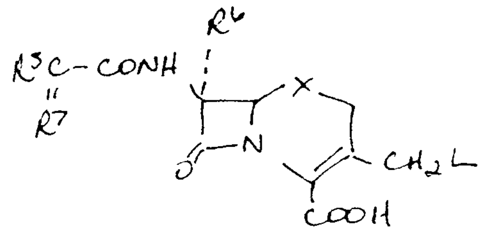

wherein $R^5$, $R^6$ and $R^7$ are as hereinbefore defined and L is a leaving group, with a source of -Y-Q wherein Y and Q are as hereinbefore defined;

(b) for preparing a compound wherein Y is a group -V-Y'-, reacting a cephalosporin compound of the formula:

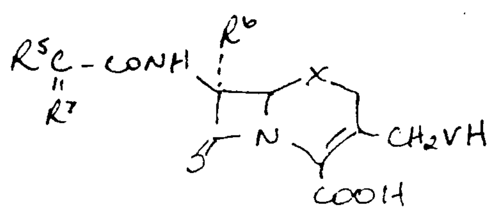

with a source of -Y'-Q wherein $R^5$, $R^6$, $R^7$, V, Y' and Q are as hereinabove defined;

(c) reacting a compound of the formula (VII) with a compound of the formula (VIII) or a reactive derivative thereof:

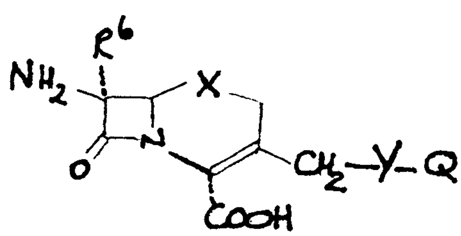

(VII)

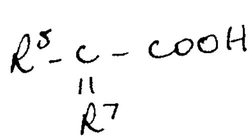

(VIII)

wherein $R^5$, $R^6$, $R^7$, X, Y and Q are as hereinbefore defined; or

d) for compounds of the formula (IV) wherein $R^7$ is a group $= NOR^8$, reacting a compound of the formula (IX):

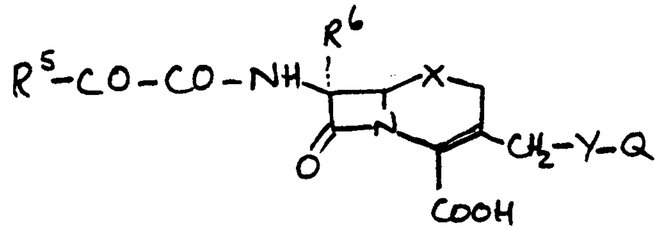

(IX)

wherein $R^5$, $R^6$, X, Y and Q are as hereinbefore defined, with a compound of the formula: $R^8ONH_2$ wherein $R^8$ is as hereinbefore defined; or

e) for compounds of the formula (IV) wherein $R^7$ is a group $=NOR^8$ and $R^8$ is other than hydrogen, reacting a compound of the formula (IV) as hereinbefore defined wherein $R^7$ is a group =NOH with a compound of the formula (X):

$L^1$ - $R^{16}$ (X)

wherein $L^1$ is a leaving group and $R^{16}$ is a group $R^8$ other than hydrogen; or

f) for compounds of the formula (IV) forming a group $R^5$ by cyclising an appropriate precursor thereof:

wherein any functional groups are optionally protected:

and thereafter, if necessary:

i) removing any protecting group,

ii) for preparing in vivo hydrolysable esters, esterifying corresponding hydroxy groups,

iii) converting compounds wherein X is S to compounds wherein X is sulphinyl and vice versa,

iv) converting compounds wherein $R^4$ is one value to compounds wherein $R^4$ has another value,

v) forming a pharmaceutically acceptable salt.

In the reaction between a cephalosporin compound having a 3-position substituent of the formula: $-CH_2L$ and a source of -Y-Q, conveniently L is a leaving group such as halo for example iodo, bromo or chloro, or is $C_{1-4}$ alkanoyloxy for example acetoxy. This reaction is most suitable for forming cephalosporin compounds wherein the 3-position substituent is substituted benzyl or substituted naphthylmethyl. Typically the reaction is performed in the presence of boron trifluoride catalyst in a substantially inert solvent such as acetonitrile at a non-extreme temperature for example ambient.

The reaction between a cephalosporin compound having a 3-position substituent of the formula: $-CH_2VH$ and a source of $-Y'$-Q, may be performed in conventional manner. For example reference may be made to GB 1496757. In a preferred aspect cephalosporin compounds having a 3-position substituent of the formula: $-CH_2N(R^4)-CH_2-Q$ can be prepared by reacting a cephalosporin having a $3-CH_2NHR^4$ substituent with an aldehyde (or equivalent) of the formula: QCHO in the presence of a reducing agent for example a borohydride. Such reductive aminations can be performed in conventional manner in a substantially inert solvent for example methanol at a non-extreme temperature for example ambient.

The cephalosporin starting materials for these reactions are known from the art, or are made by methods analogous thereto. See for example GB 1496757, EP-A-127992 and EP-A-164944.

The reaction between compounds of the formulae (VII) and (VIII) is performed under conditions conventional in the cephalosporin art, for example under standard acylation conditions wherein for example the acid is activated as an acid bromide, acid chloride, anhydride or activated ester, or the reaction is performed in the presence of a coupling reagent such as dicyclohexylcarbodi-imide.

The compounds of the formula (VII) can be prepared in a manner analogous to that described for the compounds of the formula (I), with the 7-amino group being optionally protected.

The reaction between compounds of the formula (IX) and $R^8ONH_2$ is performed under conditions standard in the general chemical and/or cephalosporin art. The compounds of the formula (IX) can be prepared in a manner analogous to that described for the compounds of the formula (I).

The reaction betweem the compound of the formula (IV) wherein $R^7$ is a group =NOH and a compound of the formula (X) is performed under conditions standard in the general chemical and/or cephalosporin art.

A group $R^5$ may be formed by cyclizing an appropriate precursor. For example compounds of the formula (XI) and (XII):

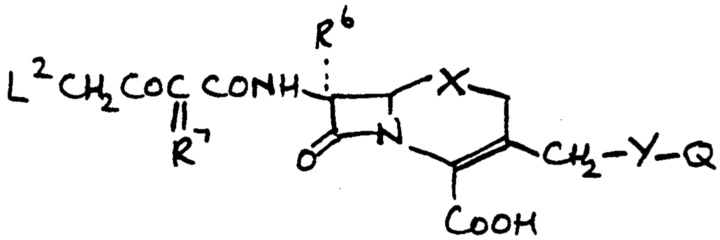

(XI)

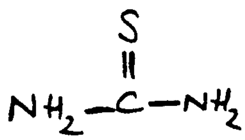

(XII)

wherein $R^7$, $R^6$, X, Y and Q are as hereinbefore defined and $L^2$ is a leaving group, may be reacted to form a 2-aminothiazol-4-yl group. A nitrogen atom of the thiourea may be optionally protected during this cyclization.

The compounds of the formula (XI) can be prepared in a manner analogous to that described for the compounds of the formula I.

The compounds of the formulae (VIII), (X) and $R^8ONH_2$ are known from, or can be made by the methods of, the general chemical and/or cephalosporin art.

The compounds of the formulae (VII), (IX) and (XI) are novel and as such form a further aspect of the present invention.

In the process of this invention any functional group can be optionally protected, if appropriate. Such protecting groups may in general be chosen from any of the groups described in the literature or known to the skilled chemist as appropriate for the protection of the group in question, and may be introduced by conventional methods.

Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Specific examples of protecting groups are given below for the sake of convenience, in which "lower" signifies that the group to which it is applied preferably has 1-4 carbon atoms. It will be understood that these examples are not exhaustive. Where specific examples of methods for the removal of protecting groups are given below these are similarly not exhaustive. The use of protecting groups and methods of deprotection not specifically mentioned is of course within the scope of the invention.

A carboxyl protecting group may be the residue of an ester-forming aliphatic or araliphatic alcohol or of an ester-forming phenol, silanol or stannanol (the said alcohol, phenol, silanol or stannanol preferably containing 1-20 carbon atoms).

Examples of carboxyl protecting groups include straight or branched chain (1-12C)alkyl groups (eg isopropyl, t-butyl); halo lower alkyl groups (eg 2-iodoethyl, 2,2,2-trichloroethyl); lower alkoxy lower alkyl groups (eg methoxymethyl, ethoxymethyl, isobutoxymethyl); lower aliphatic acyloxy lower alkyl groups, (eg acetoxymethyl, propionyloxymethyl, butyryloxymethyl, pivaloyloxymethyl); lower alkoxycarbonyloxy lower alkyl groups (eg 1-methoxy-carbonyloxyethyl, 1-ethoxycarbonyloxyethyl); aryl lower alkyl groups (eg p-methoxybenzyl, o-nitrobenzyl, p-nitrobenzyl, benzhydryl and phthalidyl); tri(lower alkyl)silyl groups (eg trimethylsilyl and t-butyldimethylsilyl); tri(lower alkyl)silyl lower alkyl groups (eg trimethylsilylethyl); and (2-6C)alkenyl groups (eg allyl and vinylethyl).

Methods particularly appropriate for the removal of carboxyl protecting groups include for example acid-, base-, metal- or enzymically-catalysed hydrolysis.

Examples of hydroxyl protecting groups include lower alkanoyl groups (eg acetyl); lower alkoxycarbonyl groups (eg t-butoxycarbonyl); halo lower alkoxycarbonyl groups (eg 2-iodoethyoxycarbonyl, 2,2,2-trichloroethoxycarbonyl); aryl lower alkoxycarbonyl groups (eg benzoyloxycarbonyl, p-methoxybenzyloxycarbonyl, onitrobenzyloxycarbonyl, p-nitrobenzyloxycarbonyl); tri lower alkylsilyl (eg trimethylsilyl, t-butyldimethylsilyl) and aryl lower alkyl (eg benzyl) groups. In addition two hydroxy groups substituted on adjacent carbon atoms, for example in the catechol moiety, may be protected in the form of a cyclic acetal such as the methylenedioxy moiety.

Examples of amino protecting groups include formyl, aralkyl groups (eg benzyl and substituted benzyl, eg p-methoxybenzyl, nitrobenzyl and 2,4-dimethoxybenzyl, and triphenylmethyl); di-p-anisylmethyl and furylmethyl groups; acyl (eg alkoxycarbonyl and aralkoxycarbonyl eg t-butoxycarbonyl and benzyloxycarbonyl), trialkylsilyl (eg trimethylsilyl and t-butyldimethylsilyl), alkylidene (eg methylidene), benzylidene and substituted benzylidene groups, and the phthalimido group.

Esterification of hydroxy groups (ie $R^1$ and $R^2$) to form in vivo hydrolysable esters is performed in conventional manner. Reduction of a cephalosporin sulphoxide to a cephalosporin and oxidation of a sulphoxide to a sulphide are performed according to methods known in the art. An example of converting one group $R^4$ to another group $R^4$ is the acylation of -NH- to $-N(COC_{1-4}alkyl)-$ or to $-N(SO_2C_{1-4}alkyl)-$.

The following biological test methods, data and Examples serve to illustrate this invention.

Antibacterial Activity

The pharmaceutically acceptable cephalosporin compounds of the present invention are useful antibacterial agents having a broad spectrum of activity in vitro against standard laboratory microorganisms, both Gram-negative and Gram-positive, which are used to screen for activity against pathogenic bacteria. The antibacterial spectrum and potency of a particular compound may be determined in a standard test system. The compounds have particularly high activity in vitro against strains of Pseudomonas aeruginosa.

The antibacterial properties of the compounds of the invention may also be demonstrated in vivo in conventional mouse protection tests.

In addition representative compounds of this invention show prolonged duration, as evidence by half-life values, in test animals.

Cephalosporin derivatives have generally been found to be relatively non-toxic to warm-blooded animals, and this generalisation holds true for the compounds of the present invention. Compounds representative of the present invention were administered to mice at doses in excess of those required to afford protection against bacterial infections, and no overt toxic symptoms or side effects attributable to the administered compounds were noted.

The following results were obtained for representative compounds on a standard in vitro test system using Isosensitest agar medium. The antibacterial activity is described in terms of the minimum inhibitory concentration (M1C) determined by the agar-dilution technique with an inoculum size of $10^4$ CFU/spot.

| ORGANISM | MIC (µl/ml) | | | |
|---|---|---|---|---|
| | EXAMPLE | | | |
| | 3 | 6 | 9 | 20 |
| P.aeruginosa PU21 (A8101028) | 4 | 0.25 | 0.25 | 2 |
| Ent. cloacae P99 (A8401054) | 4 | 2 | 4 | 8 |
| Serr.marcesens (A8421003) | 0.5 | 0.25 | NA | 4 |
| Pr.morganii (A8433001) | 2 | 0.25 | 1 | 16 |
| Kleb.aerogenes (A8391027) | 0.125 | 0.06 | 0.06 | 4 |
| E. coli DCO (A8341098) | 0.125 | 0.06 | 0.125 | 1 |
| St.aureus 147N (A8601052) | 4 | 16 | 16 | 8 |

Continued.........

.....Continued.......

| ORGANISM | MIC (µl/ml) | | | |
|---|---|---|---|---|
| | EXAMPLE | | | |
| | 3 | 6 | 9 | 20 |
| S.dublin (A8369001) | 2 | 0.25 | 0.25 | 8 |
| Strep.pyogenes (A681018) | 0.008 | 0.125 | 0.25 | NA |

In the following Examples the following abbreviations are used:

AcOH = acetic acid
DMF = dimethylformamide
DMSO = dimethylsulphoxide
EtOAc = ethyl acetate
EtOH = ethanol
HPLC = high performance liquid chromatography
MeOH = methanol
NMR = nuclear magnetic resonance spectroscopy
TEA = triethylamine
TFA = trifluoroacetic acid

The NMR spectra are taken at 90 MHz and are quoted in terms of delta values in parts per million (ppm) with reference to tetramethylsilane (delta = 0). The solvent used was $DMSOd_6/CD_3COOD/TFA$ except where otherwise indicated. In the quotation of NMR data s = singlet, d = doublet, t = triplet, q = quartet, m = multiplet, br = broad.

## Examples 1 - 7

The following general procedure was used for the preparation of the compounds of Example 1-7, of which particulars are given in Tables I and II.

To a suspension of the appropriate 7-substituted-3-aminomethyl-ceph-3-em-4-carboxylic acid (0.5 mmole) in MeOH (15 ml) containing a few drops of water, and maintained at pH 5.5-6.0 was added 3,4-dihydroxybenzaldehyde (0.55 mmole) and then sodium cyanoborohydride (0.5 mmole).

The pH was maintained at pH 5.5-6.0 by addition of sodium bicarbonate or acetic acid as appropriate, and further portions of sodium cyanoborohydride were added over 4 hours until none of the cephalosporin starting material remained (monitored by HPLC). The solvents were evaporated and the mixture purified on a Diaion HP20SS resin column using $MeOH/H_2O$ mixtures of increasing proportions of MeOH and containing AcOH (1%). Evaporation and freeze drying of the appropriate fractions gave the product in the yield indicated.

Particulars of the compounds prepared, and the yields obtained, are given in Table I. NMR characterising data is given in Table II.

Table I

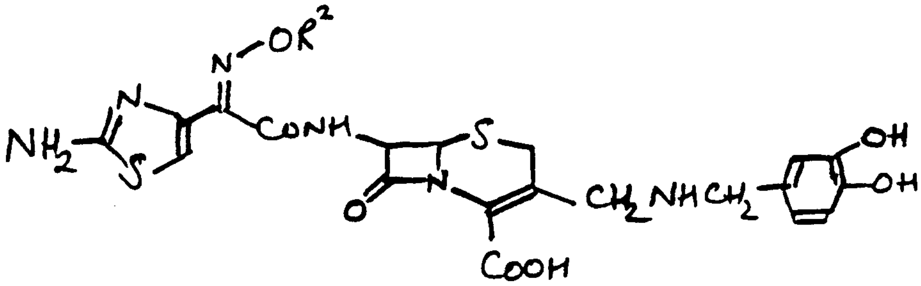

Table I continued.......

| Example No. | R2 | Yield (%) | Footnotes |
|---|---|---|---|
| 1 | $-C_2H_5$ | 27 | |
| 2 | $-CH_2CH_2F$ | 40 | |
| 3 | $-CH_2CH_2Cl$ | 57 | |
| 4 | $-CH_3$ | 25 | |
| 5 | $-CH_2CF_3$ | 33 | |
| 6 | $CH_3$<br>\|<br>— C — COOH<br>\|<br>$CH_3$ | 13 | |
| 7 | $-CH_2-COOH$ | 48 | 1 |

Footnotes

1. The general method indicated was used to prepare the diacetoxy derivative using 3,4-diacetoxybenzaldehyde instead of the 3,4-dihydroxy compound and the resulting compound 7-[2-(2-aminothiazol-4-yl)-2-((Z)-carboxymethoxyimino)acetamido]-3-(3,4-diacetoxybenzyl)aminomethylceph-3-em-4-carboxylic acid (130mg) deprotected by dissolving in $H_2O$ (50ml) containing ammonium

carbonate (100mg) at pH 7–7.5. The solution was left to stand for 2 days until no cephalosporin starting material remained. The mixture was purified on a Diaion HP20SS resin column and isolated as described above.

## Table II

**NMR data for the compounds of Table I taken at 90MHz in $DMSOd_6/CD_3CO_2D/TFAd$**

| Example No. | Delta Values (ppm). |
|---|---|
| 1 | 2.5(t,3H); 3.65(m,2H); 3.8(m,2H); 4.0(m,2H); 4.25(q,2H); 5.15(d,1H); 5.85(d,1H); 6.75(s,2H); 6.90(s,1H); 7.0(s,1H). |
| 2 | 3.65(m,2H); 3.85(m,2H); 4.0(m,2H); 4.4(m,2H); 4.6(t,1H); 5.0(t,1H); 5.2(d,1H); 5.85(d,1H); 6.75(s,2H); 6.9(s,1H); 7.0(s,1H). |
| 3 | 3.65(m,2H); 3.7–4.1(m,6H); 4.4(t,2H); 5.15(d,1H); 5.85(d,1H); 6.75(s,2H); 6.9(s,1H); 7.05(s,1H). |
| 4 | 3.65(m,2H); 3.85(m,2H); 4.0(m,2H); 4.0(s,3H); 5.15(d,1H); 5.85(d,1H); 6.75(s,2H); 6.9(s,1H); 7.0(s,1H). |
| 5 | 3.65(m,2H); 3.85(m,2H); 4.0(m,2H); 4.75(q,2H); 5.2(d,1H); 5.85(d,1H); 6.8(s,2H); 6.9(s,1H); 7.05(s,1H). |
| 6 | 1.55(s,6H); 3.65(m,2H); 3.7–3.9(m,2H); 4.0(m,2H); 5.15(d,1H); 5.9(d,1H); 6.75(s,2H); 6.9(s,1H); 7.05(s,1H). |

Table II continued.......

Table II continued.......

| Example No. | Delta Values (ppm). |
|---|---|
| 7 | 3.6–4.1(m,6H); 4.75(s,2H); 5.2(d,1H); 5.9(d,1H); 6.8(s,2H); 6.85(s,1H); 7.05(s,1H). |

## Example 8

3-(3,4-Dihydroxybenzylaminomethyl)-7-[2-(aminothiazol-4-yl)-2-((Z)-1-carboxycyclopentyloxyimino)-acetamido]ceph-3-em-4-carboxylic acid

To a suspension of 3-aminomethyl-7-[2-(aminothiazol-4-yl)-2-((Z)-1-carboxycyclopentyloxyimino)-acetamido]ceph-3-em-4-carboxylic acid (153mg) in water (10ml) with sufficient triethylamine to obtain a solution (pH 7.5) was added 3,4-dihydroxybenzaldehyde (41mg) and then sodium cyanoborohydride (19mg). The pH was adjusted and further maintained at pH 5.4 and further portions (x4) of sodium cyanoborohydride and 3,4-dihydroxybenzaldehyde added over 24 hours until no cephalosporin starting material remained. The aqueous solution was washed three times with ether and concentrated, and the crude mixture purified by HPLC on an octadecylsilane column using MeOH/water/AcOH, 20-30:79-69:1 as eluant. Evaporation and freeze drying yielded 80mg (48%) of the title compound, NMR in $DMSOd_6$ + $CD_3CO_2D$ + $TFA_d$: 1.81(s,4H); 2.15(m,4H); 3.4 to 4.2(m,6H); 5.17(d.1H); 5.9(d,1H); 6.76(br.s,2H); 6.89-(br.s,1H); 7.04(s,1H).

## Examples 9 - 20

The following general procedure was used for the preparation of the compounds of Examples 9-20, of which particulars are given in Tables III and IV.

To a solution of 7-amino-3-acetoxymethylceph-3-em-4-carboxylic acid (7-ACA) (1 mmole) in acetonitrile/$BF_3.ET_2O$ (4/1) at room temperature, was added the appropriate dihydroxy benzene or naphthalene derivative (1 mmole). Stirring was continued at room temperature for 2 to 3 hours, the progress of the reaction being followed by analytical HPLC. At the end of the reaction the solvents were evaporated and the crude product purified by medium pressure chromatography on a Diaion HP20SS column, using MeOH/water with 1% AcOH, the percentage of MeOH being slowly increased from 0 to 100%.

The 7-amino cephalosporin intermediate thus produced was dissolved in $CH_2Cl_2$ with 4-5 equivalents of bis-trimethylsilylacetamide (BSA) at room temperature under argon, several hours being necessary to achieve complete dissolution. To this solution was added a solution of the appropriate 7-side chain carboxylic acid (carboxy groups on the oxime protected as the t-butoxy derivative and the aminothiazolyl protected as the trityl derivative) in the form of the acid chloride or an active ester (eg with N-hydroxybenzotriazole (HOBT)). The mixture was shaken at room temperature for several hours, the solvent evaporated and the product treated with TFA/water at room temperature for two hours to remove all protecting groups. The compound was then purified by HPLC or medium pressure chromatography.

Particulars of the compounds prepared, and the yields obtained, are given in Table III. NMR characterising data is given in Table IV.

**Table III**

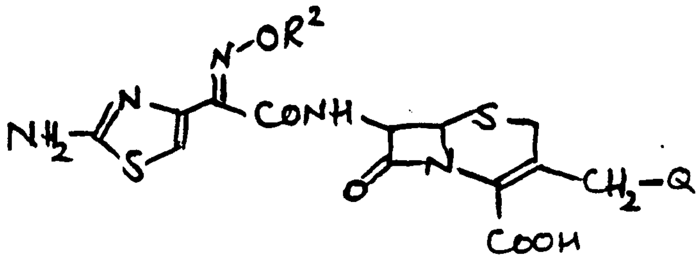

Table III continued.......

| Example No. | R2 | Q | Yield (%) |
|---|---|---|---|
| 9 | 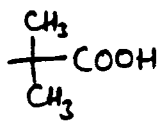 | 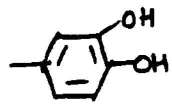 | 22 |
| 10 | $CH_3$ | 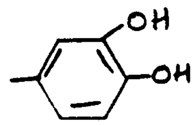 | 30 |
| 11 | 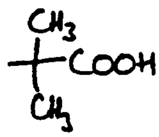 | 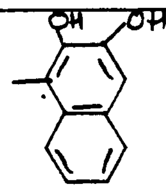 | 17 |
| 12 | $CH_3$ | 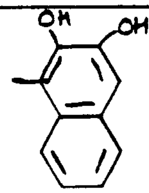 | 20 |
| 13 | 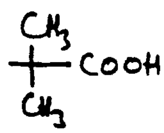 | 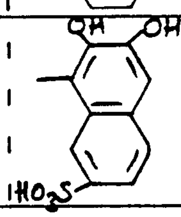 | 62 |
| 14 | 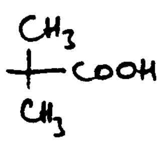 | 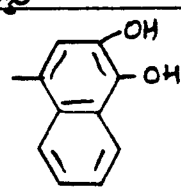 | 10 |
| 15 | $-C_2H_5$ | 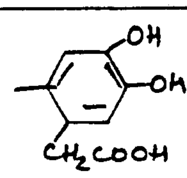 | 37 |

Table III continued........

Table III Continued..........

| Example No. | R2 | Q | Yield (%) |
|---|---|---|---|
| 16 | $-C_2H_5$ | 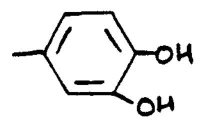 | 24 |
| 17 | $-C_2H_5$ | 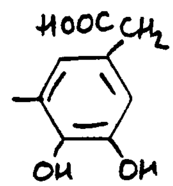 | 20 |
| 18 | $-C_2H_5$ | 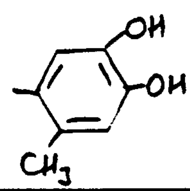 | 21 |
| 19 | $-C_2H_5$ | 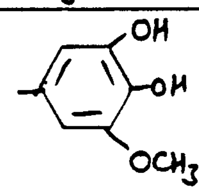 | 12 |
| 20 | $-C_2H_5$ | 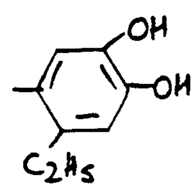 | 42 |

**Table IV**

NMR data for the compounds of Table III taken at 90MHz in $DMSOd_6$/AcOD/TFA.

| Example No. | Delta values (ppm) |
|---|---|
| 9 | 1.52(s,6H), 3.35 and 3.5(2d,2H), 3.2–4.1(m,2H); 5.17(d,1H); 5.77(d,1H); 6.5–6.9(m,3H); 7.05(s,1H). |

Table IV Contiued..........

Table IV continued........

| Example No. | Delta values (ppm) |
|---|---|
| 10 | 3.2 and 3.5(2d,2H); 3.3–4.1(m,2H); 4.0(s,3H); 5.17(d,1H); 5.7(d,1H); 6.5–6.85(m,3H); 7.0(s,1H). |
| 11 | 1.5(s,6H); 3.14(m,2H); 4.4(m,2H); 5.14(d,1H); 5.7(d,1H); 7.05(s,1H); 7.05–8(m,5H). |
| 12 | 3.1(m,2H); 4(s,3H); 4.4(m,2H); 5.1(d,1H); 5.6(d,1H); 7(s,1H); 7.1(s,1H); 7.1–8(m,4H). |
| 13 | 1.5(s,6H); 3.1(m,2H); 4.4(m,2H); 5.1(d,1H); 5.7(d,1H); 7.05(s,1H); 7.15(s,1H); 7.6(m,2H); 8.2(s,1H). |
| 14 | 1.5(s,6H); 3.1 and 3.4(2d,2H); 4.1–4.3(m,2H); 5.15(d,1H); 5.75(d,1H); 7.08(m,2H); 7.2–8.3(m,4H). |
| 15 | 1.27(t,3H); 3.1 and 3.4(2d,2H); 3.44(m,2H); 3.5–3.8(m,2H); 4.24(q,2H); 5.15(d,1H); 5.75(d,1H); 6.6(s,2H); 7.0(s,1H). |
| 16 | 1.25(t,3H); 3.15 and 3.45(2d,2H); 3.2–4(m,2H); 4.2(q,2H); 5.15(d,1H); 5.7(d,1H); 6.4–6.8(m,2H); 6.7(s,1H); 7.0(s,1H). |
| 17 | 1.15(t,3H); 3.1–3.3(m,2H); 3.75(m,2H); 4.2(q,2H); 5.15(d,1H); 5.65(d,1H); 6.5(s,1H); 6.6(s,1H); 6.95(s,1H). |

Table IV continued.......

Table IV continued........

| Example No. | Delta values (ppm) |
|---|---|
| 18 | 1.15(t,3H); 2.05(s,3H); 3.1 and 3.35(2d,2H); 3.5 and 3.75(2d,2H); 4.24(q,2H); 5.15(d,1H); 5.7(d,1H); 6.55(2s,2H); 7.0(s,1H). |
| 19 | 1.25(t,3H); 3.7(s,3H); 3–4(m,4H); 4.24(q,2H); 5.1(d,1H); 5.65(d,1H); 6.45(s,2H); 6.95(s,1H). |
| 20* | 1.05(t,3H); 1.26(t,3H); 2.45(q,2H); 3.1 and 3.4(2d,2H); 3.5 and 3.8(2d,2H); 4.24(q,2H); 5.15(d,1H); 5.7(d,1H); 6.55(s,2H); 7.0(s,1H). |

* Solvent = $CF_3CO_2D/CD_3CO_2D/DMSOd_6$

**Example 21**

7-[2-(2-Aminothiazol-4-yl)-2-((Z)-ethoxyimino)acetamido-3-(N-3,4-diacetoxybenzyl)aminomethyl]ceph-3-em-4-carboxylic acid (190mg, 0.3mmol) was suspended in acetonitrile (4ml), the suspension was cooled to 0 ° C, and treated, in quick succession, with triethylamine (61mg, 83µl, 0.6 mmol) and acetyl chloride (24mg, 22µl, 0.3 mmol). The mixture was stirred at 0 ° for 1 hour, the acetonitrile removed under reduced pressure and the residue so obtained applied to a Diaion HP 20 SS resin column in $H_2O$/DMF. The column was developed by gradient elution ($H_2O$ - 20% $CH_3CN/H_2O$), the appropriate fractions combined, acetonitrile removed and 7-[2-(2-aminothiazol-4-yl)-2((Z)-ethoxyimino)acetamido-3-(N-acetyl-N-3,4-diacetoxybenzyl)-aminomethyl]ceph-3-em-4-carboxylic acid obtained (in 36% yield) by freeze-drying; NMR ($d_6$DMSO,$d_4$HOAC) 1.18(t,3H); 2.02, 2.12(s,s,3H, 2 rotamers); 2.21(s, 6H); 3.30(d, 1H); 3.45(d,d, 1H), 4.09(q, 2H); 4.2-4.65(m, 2H); 5.03, 5.06(d,d, 1H, 2 rotamers), 5.76(d,d, 1H, 2 rotamers), 6,61(s, 1H), 7-7.25(m, 3H).

**Example 22**

7-[2-(2-Aminothiazol-4-yl)-2-((Z)-ethoxyimino)acetamido-3-(N-3,4-diacetoxybenzyl)aminomethyl]ceph-3-em-4-carboxylic acid(250mg, 0.4mmol) was suspended in acetonitrile (5ml), the suspension cooled to 0 ° C, treated with triethylamine (80mg, 109µl, 0.8 mmol) and, subsequently, with methanesulphonylchloride (45mg, 31µl, 0.4 mmol). The mixture was stirred for 10 minutes before adding further triethylamine (80mg, 0.8 mmol). After stirring for a further 1 1/2 hours the mixture was poured on to $H_2O$ (75ml) and the solution purified by chromatography on Diaion HP 20 SS resin (gradient elution, $H_2O$ - 20% $CH_3CN/H_2O$). Appropriate fractions were combined, acetonitrile removed under reduced pressure and triethylammonium 7-[2-(2-aminothiazol-4-yl)-2-((Z)-ethoxyimino)acetamido-3-(N-3,4-diacetoxybenzyl-N-methanesulphonyl)-aminomethyl]-ceph-3-em-4-carboxylate isolated by freeze-drying (48% yield); NMR ($d_6$DMSO,$d_4$HOAc) 1.16(t, 9H); 1.2(t, 3H); 2.2(s, 3H); 2.22(s, 3H); 2.97(s, 3H); 3.08(q, 6H); 3.24(s, 2H); 4.08(q, 2H); 4.19(d, 1H); 4.20(br, 2H); 4.45(d, 1H); 4.77(d, 1H); 5.68(d, 1H); 6.7(s, 1H); 7.1-7.3(m, 3H).

## Claims

### Claims for the following Contracting States : AT, BE, CH, DE, FR, GB, IT, LI, LU, NL, SE

1. A cephalosporin compound of the formula (IV):

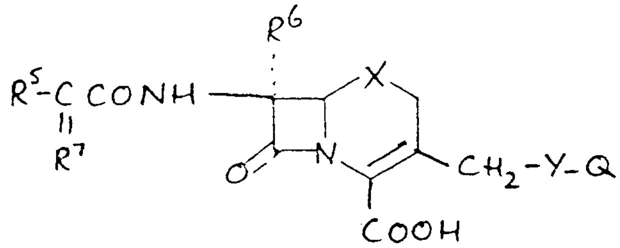

(IV)

or a salt or ester thereof; wherein:
X is sulphur, oxygen, methylene or sulphinyl;
$R^6$ is hydrogen, methoxy or formamido;

$R^5$ is 2-aminothiazol-4-yl or 2-aminooxazol-4-yl each optionally substituted in the 5-position by fluorine, chlorine or bromine, or $R^5$ is 5-aminoisothiazol-3-yl, 5-amino-1,2,4-thiadiazol-3-yl, 3-aminopyrazol-5-yl, 3-aminopyrazol-4-yl, 2-aminopyrimidin-5-yl, 2-aminopyrid-6-yl, 4-aminopyrimidin-2-yl, 2-amino-1,3,4-thiadiazol-5-yl or 5-amino-1-methyl-1,2,4-triazol-3-yl;

$R^7$ is of the formula =N.O.$R^8$ (having the syn configuration about the double bond) wherein $R^8$ is hydrogen, (1-6C)alkyl, (3-8C)cycloalkyl, (1-3C)alkyl(3-6C)cycloalkyl, (3-6C)cycloalkyl(1-3C)alkyl, (3-6C)-alkenyl, optionally substituted by carboxy, (5-8C)cycloalkenyl, (3-6C)alkynyl, (2-5C)alkylcarbamoyl, phenylcarbamoyl, benzylcarbamoyl, (1- 4C)alkylcarbamoyl(1-4C)alkyl, di(1-4C)alkylcarbamoyl(1-4C)-alkyl, (1-4C)haloalkylcarbamoyl(1-4C)alkyl, (1-3C)haloalkyl, (2- 6C)hydroxyalkyl, (1-4C)alkoxy(2-4C)alkyl, (1-4C)alkylthio(2-4C)alkyl, (1-4C)alkanesulphinyl(1-4C)alkyl, (1-4C)alkanesulphonyl(1-4C)alkyl, (2-6C)-aminoalkyl, (1-4C)alkylamino(1-6C)alkyl, (2-8C)dialkylamino(2-6C)alkyl, (1-5C)cyanoalkyl, 3-amino-3-carboxypropyl, 2-(amidinothio)ethyl, 2-(N-aminoamidinothio)ethyl, tetrahydropyran-2-yl, thietan-3-yl, 2-oxopyrrolidinyl, or 2-oxotetrahydrofuranyl, or $R^8$ is of the formula V:-

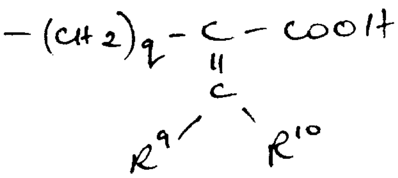

(V)

wherein q is one or two and $R^9$ and $R^{10}$ are independently hydrogen or $C_{1-4}$alkyl; or $R^8$ is of the formula VI:-

$-CR^{11}R^{12}-(CH_2)_r-COR^{13}$ (VI)

wherein r is 0-3, $R^{11}$ is hydrogen, (1-3C)alkyl or methylthio, $R^{12}$ is hydrogen (1-3C)alkyl, (3-7C)-cycloalkyl, cyano, carboxy, (2-5C)carboxyalkyl or methanesulphonylamino, or $R^{11}$ and $R^{12}$ are joined to form, together with the carbon to which they are attached, a (3-7C)carbocyclic ring, and $R^{13}$ is hydroxy, amino, (1-4C)alkoxy, (1-4C) alkylamino or of the formula $NHOR^{14}$ in which $R^{14}$ is hydrogen or (1-4C)-alkyl;

or $R^7$ may be of the formula =CH.$R^{15}$ wherein $R^{15}$ is hydrogen, halogen, (1-6C)alkyl, (3-7C)-cycloalkyl, (2-6C)alkenyl, (3-7C)cycloalkenyl, phenyl or benzyl;

Q is:

(i) a benzene ring (optionally fused to a further benzene ring so forming a naphthyl group or optionally fused to a 5 or 6 membered heterocyclic aromatic group containing 1, 2 or 3 heteroatoms selected from nitrogen, oxygen and sulphur), said benzene ring being substituted by groups $R^1$ and $R^2$ which are ortho with respect to one another, wherein $R^1$ is hydroxy and $R^2$ is hydroxy:

(ii) a group of the formula (II):

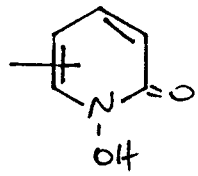

or;
(iii) a group of the formula (III):

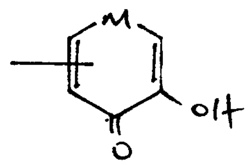

wherein M is oxygen or a group $NR^3$
wherein $R^3$ is hydrogen or $C_{1-4}$ alkyl:

ring Q (or, in the case wherein ring Q is a benzene ring fused to another benzene ring, either benzene ring) is optionally further substituted by $C_{1-4}$ alkyl, halo, hydroxy, hydroxy $C_{1-4}$ alkyl, cyano, trifluoromethyl, nitro, amino, $C_{1-4}$ alkylamino di-$C_{1-4}$ alkylamino, amino $C_{1-4}$ alkyl, $C_{1-4}$ alkylamino $C_{1-4}$ alkyl, di-$C_{1-4}$ alkylamino $C_{1-4}$ alkyl, $C_{1-4}$ alkanoyl, $C_{1-4}$ alkoxy, $C_{1-4}$ alkylthio, $C_{1-4}$ alkanoyloxy, carbamoyl, $C_{1-4}$ alkylcarbamoyl, di-$C_{1-4}$ alkyl carbamoyl, carboxy, carboxy $C_{1-4}$ alkyl, sulpho, sulpho $C_{1-4}$ alkyl, $C_{1-4}$ alkanesulphonamido, $C_{1-4}$ alkoxycarbonyl, $C_{1-4}$ alkanoylamino, nitroso, thioureido, amidino, ammonium, mono- , di- or tri-$C_{1-4}$ alkylammonium or pyridinium, or a 5-membered heterocyclic ring containing 1 to 4 heteroatoms selected from oxygen, nitrogen and sulphur which is optionally substituted by 1, 2 or 3 $C_{1-4}$ alkyl or $C_{1-4}$ alkoxy groups;

Y, which links into the benzene ring or the ring of formula (II) or (III), is a covalent bond or is a group -V-Y'- wherein V is oxygen, sulphur or a group -$NR^4$- and -Y'- is a covalent bond or $C_{1-4}$ alkylene, wherein $R^4$ is hydrogen, $C_{1-4}$ alkyl optionally substituted by any one of halo, hydroxy, $C_{1-4}$ alkoxy, carboxy, amino, cyano, $C_{1-4}$ alkanoylamino or phenyl or $R^4$ is $C_{2-6}$ alkenyl $C_{1-4}$ alkanesulphonyl or $C_{1-4}$ alkanoyl.

**2.** A compound according to claim 1 wherein Q is a benzene ring (optionally fused to a further benzene ring so forming a naphthyl group) substituted by groups $R^1$ and $R^2$ which are <u>ortho</u> to one another wherein $R^1$ and $R^2$ are independently hydroxy or an in vivo hydrolysable ester thereof said benzene ring or rings being optionally further substituted.

**3.** A compound according to either claim 1 or claim 2 wherein -Y- is a covalent bond.

**4.** A compound according to either claim 1 or claim 2 wherein -Y- is $-N(R^4)CH_2-$.

**5.** A compound according to claim 4 wherein $R^4$ is hydrogen.

**6.** A compound according to claim 1 which is 3-(3,4-dihydroxybenzylaminomethyl)-7-[2-(2-aminothiazol-4-yl)-2-((Z)-1-carboxy-1-methylethoxyimino)acetamido]ceph-3-em-4-carboxylic acid; or 3-(3,4-dihydroxybenzyl)-7-[2-(2-aminothiazol-4-yl)-2-((Z)-1-carboxy-1-methylethoxyimino)acetamido]ceph-3-em-4-carboxylic acid.

**7.** A pharmaceutical composition which comprises a compound according to any one of claims 1 to 6 and a pharmaceutically acceptable carrier.

**8.** A process for preparing a compound according to claim 1 which process comprises:

(a) reacting a cephalosporin compound of the formula:

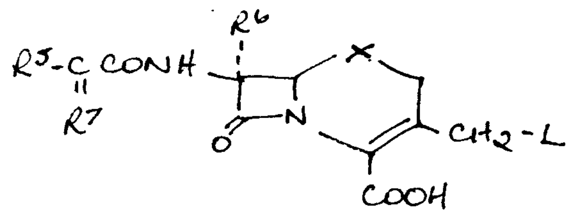

wherein $R^5$, $R^6$ and $R^7$ are as hereinbefore defined and L is a leaving group, with a source of -Y-Q wherein Y and Q are as hereinbefore defined;

(b) for preparing a compound wherein Y is a group -V-Y'-, reacting a cephalosporin compound of the formula:

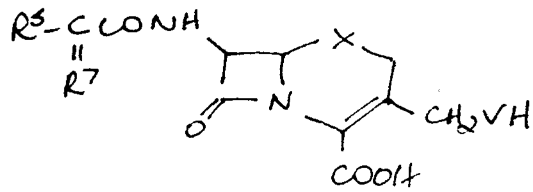

with a source of -Y'-Q wherein $R^5$, $R^6$, $R^7$, V, Y' and Q are as hereinbefore defined;

(c) for preparing compounds of the formula (IV) as defined in claim 1, reacting a compound of the formula (VII) with a compound of the formula (VIII) or a reactive derivative thereof:

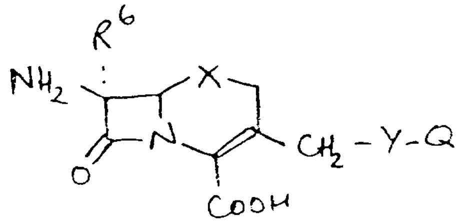

(VII)

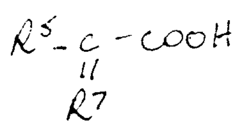

(VIII)

wherein $R^5$, $R^6$, $R^7$ and X are as defined in claim 1 or

d) for compounds of the formula (IV) wherein $R^7$ is a group = $NOR^8$, reacting a compound of the formula (IX):

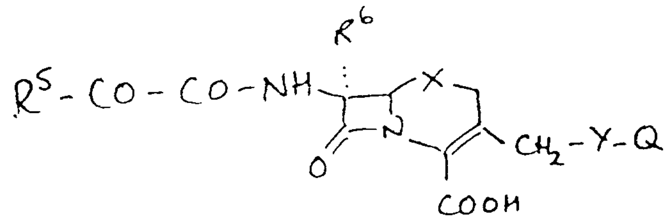

(IX)

wherein $R^5$, $R^6$, X, Y and Q are as hereinbefore defined, with a compound of the formula: $R^8ONH_2$ wherein $R^8$ is as defined in claim 6; or

e) for compounds of the formula (IV) wherein $R^7$ is a group $=NOR^8$ and $R^8$ is other than hydrogen, reacting a compound of the formula (IV) as hereinbefore defined wherein $R^7$ is a group $=NOH$ with a compound of the formula (X):

$L^1 - R^{16}$ (X)

wherein $L^1$ is a leaving group and $R^{16}$ is a group $R^8$ other than hydrogen; or

f) for compounds of the formula (IV) forming a group $R^5$ by cyclising an appropriate precursor thereof:

wherein any functional groups are optionally protected: and thereafter, if necessary:

i) removing any protecting group,
ii) for preparing in vivo hydrolysable esters, esterifying corresponding hydroxy groups,
iii) converting compounds wherein X is S to compounds wherein X is sulphinyl and vice versa,
iv) converting compounds wherein $R^4$ is one value to compounds wherein $R^4$ has another value,
v) forming a pharmaceutically acceptable salt.

**9.** A compound of the formula (VII or (IX) as defined in claim 8, or a compound of the formula (XI):

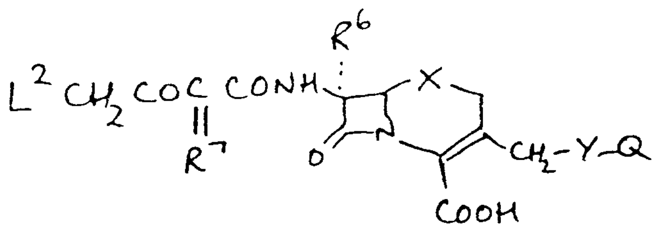

(XI)

wherein $L^2$ is a leaving group and $R^7$, $R^6$, X, Y and Q are as defined in claim 8.

**Claims for the following Contracting States : ES, GR**

**1.** A process for preparing a cephalosporin compound of the formula (IV):

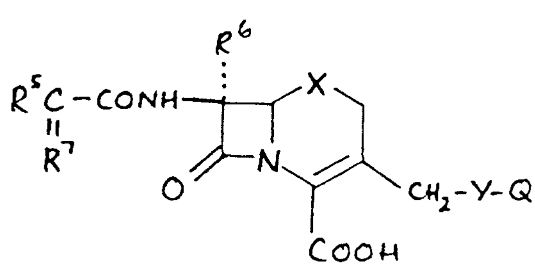

(IV)

a salt or ester thereof; wherein:
X is sulphur, oxygen, methylene or sulphinyl;

$R^6$ is hydrogen, methoxy or formamido;

$R^5$ is 2-aminothiazol-4-yl or 2-aminooxazol-4-yl each optionally substituted in the 5-position by fluorine, chlorine or bromine, or $R^5$ is 5-aminoisothiazol-3-yl, 5-amino-1,2,4-thiadiazol-3-yl, 3-aminopyrazol-5-yl, 3-aminopyrazol-4-yl, 2-aminopyrimidin-5-yl, 2-aminopyrid-6-yl, 4-aminopyrimidin-2-yl, 2-amino-1,3,4-thiadiazol-5-yl or 5-amino-1-methyl-1,2,4-triazol-3-yl;

$R^7$ is of the formula =N.O.$R^8$ (having the syn configuration about the double bond) wherein $R^8$ is hydrogen, (1-6C)alkyl, (3-8C)cycloalkyl, (1-3C)alkyl(3-6C)cycloalkyl, (3-6C)cycloalkyl(1-3C)alkyl, (3-6C)-alkenyl, optionally substituted by carboxy, (5-8C)cycloalkenyl, (3-6C)alkynyl, (2-5C)alkylcarbamoyl, phenylcarbamoyl, benzylcarbamoyl, (1- 4C)alkylcarbamoyl(1-4C)alkyl, di(1-4C)alkylcarbamoyl(1-4C)-alkyl, (1-4C)haloalkylcarbamoyl(1-4C)alkyl, (1-3C)haloalkyl, (2- 6C)hydroxyalkyl, (1-4C)alkoxy(2-4C)alkyl, (1-4C)alkylthio(2-4C)alkyl, (1-4C)alkanesulphinyl(1-4C)alkyl, (1-4C)alkanesulphonyl(1-4C)alkyl, (2-6C)-aminoalkyl, (1-4C)alkylamino(1-6C)alkyl, (2-8C)dialkylamino(2-6C)alkyl, (1-5C)cyanoalkyl, 3-amino-3-carboxypropyl, 2-(amidinothio)ethyl, 2-(N-aminoamidinothio)ethyl, tetrahydropyran-2-yl, thietan-3-yl, 2-oxopyrrolidinyl, or 2-oxotetrahydrofuranyl, or $R^8$ is of the formula V:-

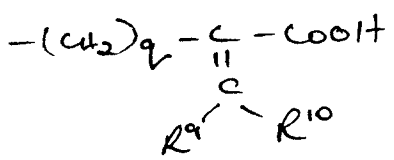 (V)

wherein q is one or two and $R^9$ and $R^{10}$ are independently hydrogen or $C_{1-4}$alkyl; or $R^8$ is of the formula VI:-

$-CR^{11}R^{12}-(CH_2)_r-COR^{13}$ (VI)

wherein r is 0-3, $R^{11}$ is hydrogen, (1-3C)alkyl or methylthio, $R^{12}$ is hydrogen (1-3C)alkyl, (3-7C)-cycloalkyl, cyano, carboxy, (2-5C)carboxyalkyl or methanesulphonylamino, or $R^{11}$ and $R^{12}$ are joined to form, together with the carbon to which they are attached, a (3-7C)carbocyclic ring, and $R^{13}$ is hydroxy, amino, (1-4C)alkoxy, (1-4C) alkylamino or of the formula NHOR$^{14}$ in which $R^{14}$ is hydrogen or (1-4C)-alkyl;

or $R^7$ may be of the formula =CH.$R^{15}$ wherein $R^{15}$ is hydrogen, halogen, (1-6C)alkyl, (3-7C)-cycloalkyl, (2-6C)alkenyl, (3-7C)cycloalkenyl, phenyl or benzyl;

Q is:

(i) a benzene ring (optionally fused to a further benzene ring so forming a naphthyl group or optionally fused to a 5 or 6 membered heterocyclic aromatic group containing 1, 2 or 3 heteroatoms selected from nitrogen, oxygen and sulphur), said benzene ring being substituted by groups $R^1$ and $R^2$ which are ortho with respect to one another, wherein $R^1$ is hydroxy and $R^2$ is hydroxy;

(ii) a group of the formula (II):

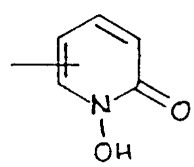

or;

(iii) a group of the formula (III):

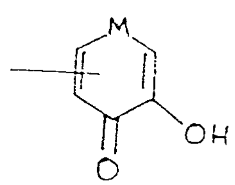

wherein M is oxygen or a group $NR^3$
wherein $R^3$ is hydrogen or $C_{1-4}$ alkyl:

ring Q (or, in the case wherein ring Q is a benzene ring fused to another benzene ring, either benzene ring) is optionally further substituted by $C_{1-4}$ alkyl, halo, hydroxy, hydroxy $C_{1-4}$ alkyl, cyano, trifluoromethyl, nitro, amino, $C_{1-4}$ alkylamino, di-$C_{1-4}$ alkylamino, amino $C_{1-4}$ alkyl, $C_{1-4}$ alkylamino $C_{1-4}$ alkyl, di-$C_{1-4}$ alkylamino $C_{1-4}$ alkyl, $C_{1-4}$ alkanoyl, $C_{1-4}$ alkoxy, $C_{1-4}$ alkylthio, $C_{1-4}$ alkanoyloxy, carbamoyl, $C_{1-4}$ alkylcarbamoyl di-$C_{1-4}$ alkyl carbamoyl, carboxy, carboxy $C_{1-4}$ alkyl, sulpho, sulpho $C_{1-4}$ alkyl, $C_{1-4}$ alkanesulphonamido, $C_{1-4}$ alkoxycarbonyl, $C_{1-4}$ alkanoylamino, nitroso, thioureido, amidino, ammonium, mono- , di- or tri-$C_{1-4}$ alkylammonium or pyridinium, or a 5-membered heterocyclic ring containing 1 to 4 heteroatoms selected from oxygen, nitrogen and sulphur which is optionally substituted by 1, 2 or 3 $C_{1-4}$ alkyl or $C_{1-4}$ alkoxy groups;

Y, which links into the benzene ring or the ring of formula (II) or (III), is a covalent bond or is a group -V-Y'- wherein V is oxygen, sulphur or a group $-NR^4-$ and -Y'- is a covalent bond or $C_{1-4}$ alkylene, wherein $R^4$ is hydrogen, $C_{1-4}$ alkyl optionally substituted by any one of halo, hydroxy, $C_{1-4}$ alkoxy, carboxy, amino, cyano, $C_{1-4}$ alkanoylamino or phenyl or $R^4$ is $C_{2-6}$ alkenyl $C_{1-4}$ alkanesulphonyl or $C_{1-4}$ alkanoyl;

which processes comprises:

(a) reacting a cephalosporin compound of the formula:

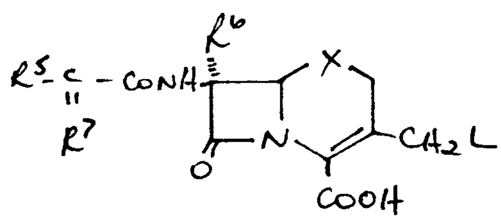

wherein $R^5$, $R^6$ and $R^7$ are as hereinbefore defined and L is a leaving group, with a source of -Y-Q wherein Y and Q are as hereinbefore defined;

(b) for preparing a compound wherein Y is a group -V-Y'-, reacting a cephalosporin compound of the formula:

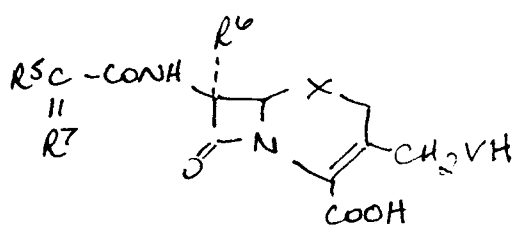

with a source of -Y'-Q wherein $R^5$, $R^6$, $R^7$, V, Y' and Q are as hereinbefore defined;

wherein any functional groups are optionally protected: and thereafter, if necessary:

i) removing any protecting group,

ii) for preparing in vivo hydrolysable esters, esterifying corresponding hydroxy groups,

iii) converting compounds wherein $R^4$ is one value to compounds wherein $R^4$ has another value,

iv) forming a pharmaceutically acceptable salt.

**2.** A process for preparing a compound of the formula (IV), wherein $R^5$, $R^6$, $R^7$, Y and Q are as defined in claim 1:

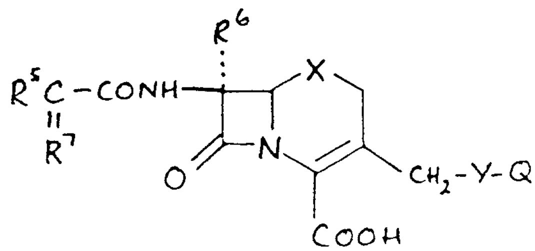

(IV)

which process comprises:
i) reacting a compound of the formula (VII) with a compound of the formula (VIII) or a reactive derivative thereof:

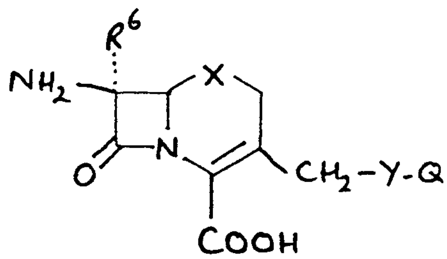

(VII)

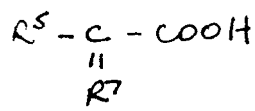

(VIII)

wherein $R^5$, $R^6$, $R^7$ and X are as defined hereinbefore or ii) for compounds of the formula (IV) wherein $R^7$ is a group $=NOR^8$, reacting a compound of the formula (IX):

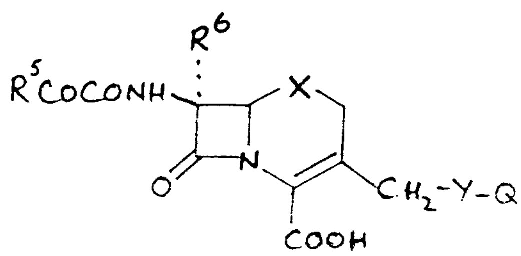

(IX)

wherein $R^5$, $R^6$, X, Y and Q are as hereinbefore defined, with a compound of the formula: $R^8ONH_2$ wherein $R^8$ is as defined hereinbefore; or
iii) for compounds of the formula (IV) wherein $R^7$ is a group $=NOR^8$ and $R^8$ is other than hydrogen, reacting a compound of the formula (IV) as hereinbefore defined wherein $R^7$ is a group $=NOH$ with a compound of the formula (X):

$L^1 - R^{16}$ (X)

wherein $L^1$ is a leaving group and $R^{16}$ is a group $R^8$ other than hydrogen; or
iv) for compounds of the formula (IV) forming a group $R^5$ by cyclising an appropriate precursor thereof: wherein any functional groups are optionally protected: and thereafter, if necessary:

i) removing any protecting group,
ii) for preparing in vivo hydrolysable esters, esterifying corresponding hydroxy groups,
iii) converting compounds wherein X is S to compounds wherein X is sulphinyl and vice versa,
iv) converting compounds wherein $R^4$ is one value to compounds wherein $R^4$ has another value,
v) forming a pharmaceutically acceptable salt.

**3.** A process according to claim 1 or claim 2 which comprises preparing a compound wherein Q is a benzene ring (optionally fused to a further benzene ring so forming a naphthyl group) substituted by groups $R^1$ and $R^2$ which are ortho to one another wherein $R^1$ and $R^2$ are hydroxy said benzene ring or rings being optionally further substituted.

**4.** A process according to any one of claims 1 to 3 for preparing a compound wherein -Y- is a covalent bond.

**5.** A process according to any one of claims 1 to 3 for preparing a compound wherein -Y- is $-N(R^4)CH_2-$.

**6.** A process according to claim 5 for preparing a compound wherein $R^4$ is hydrogen.

**7.** A process for preparing a pharmaceutical composition which comprises bringing into association a cephalosporin compound of the formula (IV) as defined in claim 1 and a pharmaceutically acceptable carrier.

**Patentansprüche**
**Patentansprüche für folgende Vertragsstaaten : AT, BE, CH, DE, FR, GB, IT, LI, LU, NL, SE**

**1.** Cephalosporin-Verbindung mit der folgenden Formel (IV):

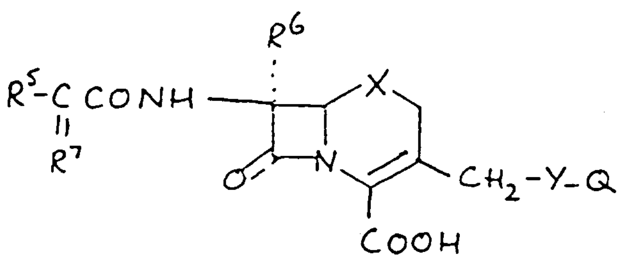

(IV)

oder ein Salz oder Ester davon; in der:
X für Schwefel, Sauerstoff, Methylen oder Sulfinyl steht;
$R^6$ für Wasserstoff, Methoxy oder Formamido steht;
$R^5$ für 2-Aminothiazol-4-yl oder 2-Aminooxazol-4-yl steht, die jeweils gegebenenfalls in der 5-Stellung durch Fluor, Chlor oder Brom substituiert sind, oder in der $R^5$ für 5-Aminoisothiazol-3-yl, 5-Amino-1,2,4-thiadiazol-3-yl, 3-Aminopyrazol-5-yl, 3-Aminopyrazol-4-yl, 2-Aminopyrimidin-5-yl, 2-Aminopyrid-6-yl, 4-Aminopyrimidin-2-yl, 2-Amino-1,3,4-thiadiazol-5-yl oder 5-Amino-1-methyl-1,2,4-triazol-3-yl steht;
$R^7$ für die Formel $=N.O.R^8$ steht (die an der Doppelbindung die syn-Konfiguration aufweist), wobei $R^8$ für folgendes steht: Wasserstoff, (1-6C)Alkyl, (3-8C)Cycloalkyl, (1-3C)Alkyl(3-6C)cycloalkyl, (3-6C)-Cycloalkyl(1-3C)alkyl, (3-6C)Alkenyl, gegebenenfalls durch Carboxy substituiert, (5-8C)Cycloalkenyl, (3-6C)Alkinyl, (2-5C)Alkylcarbamoyl, Phenylcarbamoyl, Benzylcarbamoyl, (1-4C)Alkylcarbamoyl(1-4C)alkyl, Di(1-4C)alkylcarbamoyl(1-4C)alkyl, (1-4C)Halogenalkylcarbamoyl(1-4C)alkyl, (1-3C)Halogenalkyl, (2-6C)-Hydroxyalkyl, (1-4C)Alkoxy(2-4C)alkyl, (1-4C)Alkylthio(2-4C)alkyl, (1-4C)Alkansulfinyl(1-4C)alkyl, (1-4C)-Alkansulfonyl(1-4C)alkyl, (2-6C)Aminoalkyl, (1-4C)Alkylamino(1-6C)alkyl, (2-8C)Dialkylamino(2-6C)alkyl, (1-5C)Cyanoalkyl, 3-Amino-3-carboxypropyl, 2-(Amidinothio)ethyl, 2-(N-Aminoamidinothio)ethyl, Tetrahydropyran-2-yl, Thietan-3-yl, 2-Oxopyrrolidinyl oder 2-Oxotetrahydrofuranyl; oder in der $R^8$ die Formel V hat:-

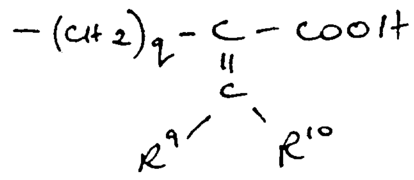

(V)

in der q für 1 oder 2 steht und $R^9$ und $R^{10}$ unabhängig voneinander für Wasserstoff oder $C_{1-4}$-Alkyl stehen; oder in der $R^8$ die Formel VI hat:-

$-CR^{11}R^{12}-(CH_2)_r-COR^{13}$ (VI)

in der r für 0 - 3 steht, $R^{11}$ für Wasserstoff, (1-3C)Alkyl oder Methylthio steht, $R^{12}$ für Wasserstoff, (1-3C)Alkyl, (3-7C)Cycloalkyl, Cyano, Carboxy, (2-5C)Carboxyalkyl oder Methansulfonylamino steht, oder in der $R^{11}$ und $R^{12}$ zusammen mit dem Kohlenstoff, an den sie gebunden sind, unter Bildung eines (3-7C)carbocyclischen Rings verbunden sind, und in der $R^{13}$ für Hydroxy, Amino, (1-4C)Alkoxy, (1-4C)-Alkylamino oder für die Formel $NHOR^{14}$ steht, wobei $R^{14}$ für Wasserstoff oder (1-4C)Alkyl steht;
oder in der $R^7$ für die Formel $=CH.R^{15}$ stehen kann, in der $R^{15}$ für Wasserstoff, Halogen, (1-6C)Alkyl, (3-7C)Cycloalkyl, (2-6C)Alkenyl, (3-7C)Cycloalkenyl, Phenyl oder Benzyl steht;
Q steht für:

(i) einen Benzol-Ring (der gegebenenfalls an einen weiteren Benzol-Ring unter Bildung einer Naphthyl-Gruppe kondensiert ist oder der gegebenenfalls an eine 5- oder 6-gliedrige heterocyclische aromatische Gruppe, die 1, 2 oder 3 aus Stickstoff, Sauerstoff und Schwefel ausgewählte Heteroatome enthält, kondensiert ist), wobei der Benzol-Ring durch Gruppen $R^1$ und $R^2$, die sich in ortho-Stellung zueinander befinden, substituiert ist, wobei $R^1$ für Hydroxy steht und $R^2$ für Hydroxy steht;
(ii) eine Gruppe mit der Formel (II):

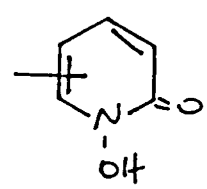

oder;
(iii) eine Gruppe mit der Formel (III):

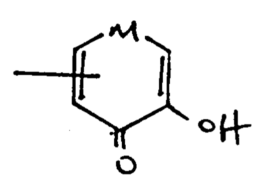

in der M für Sauerstoff oder eine Gruppe $NR^3$ steht, wobei $R^3$ für Wasserstoff oder $C_{1-4}$-Alkyl steht, wobei der Ring Q (oder jeder Benzol-Ring, falls Ring Q für einen Benzol-Ring steht, der an einen anderen Benzol-Ring kondensiert ist) gegebenenfalls mit folgendem weitersubstituiert ist: $C_{1-4}$-Alkyl, Halogen, Hydroxy, Hydroxy-$C_{1-4}$-alkyl, Cyano, Trifluormethyl, Nitro, Amino, $C_{1-4}$-Alkylamino, Di-$C_{1-4}$-alkylamino, Amino-$C_{1-4}$-alkyl, $C_{1-4}$-alkylamino-$C_{1-4}$-alkyl, Di-$C_{1-4}$-alkylamino-$C_{1-4}$-alkyl, $C_{1-4}$-Alkanoyl, $C_{1-4}$-Alkoxy, $C_{1-4}$-Alkylthio, $C_{1-4}$-Alkanoyloxy, Carbamoyl, $C_{1-4}$-Alkylcarbamoyl, Di-$C_{1-4}$-alkylcarbamoyl, Carboxy, Carboxy-$C_{1-4}$-alkyl, Sulfo, Sulfo-$C_{1-4}$-alkyl, $C_{1-4}$-Alkansulfonamido, $C_{1-4}$-Alkoxycarbonyl, $C_{1-4}$-Alkanoylamino, Nitroso, Thioureido, Amidino, Ammonium, Mono-, Di- oder Tri-$C_{1-4}$-alkylammonium oder Pyridinium, oder einem 5-gliedrigen 1 bis 4 aus Sauerstoff, Stickstoff und Schwefel ausgewählte Heteroatome enthaltenden heterocyclischen Ring, der gegebenenfalls mit 1, 2 oder 3 $C_{1-4}$-Alkyl- oder $C_{1-4}$-Alkoxy-Gruppen substituiert ist;
Y, das an den Benzol-Ring oder den Ring mit der Formel (II) oder (III) bindet, für eine kovalente

Bindung oder für eine Gruppe -V-Y'- steht, wobei V für Sauerstoff, Schwefel oder eine Gruppe $-NR^4-$ steht und -Y'-für eine kovalente Bindung oder $C_{1-4}$-Alkenylen steht, wobei $R^4$ für Wasserstoff, $C_{1-4}$-Alkyl, das gegebenenfalls mit Halogen, Hydroxy, $C_{1-4}$-Alkoxy, Carboxy, Amino, Cyano, $C_{1-4}$-Alkanoylamino oder Phenyl substituiert ist, steht, oder wobei $R^4$ für $C_{2-6}$-Alkenyl, $C_{1-4}$-Alkansulfonyl oder $C_{1-4}$-Alkanoyl steht.

2. Verbindung nach Anspruch 1, wobei Q für einen Benzol-Ring (der gegebenenfalls unter Bildung einer Naphthyl-Gruppe an einen weiteren Benzol-Ring kondensiert ist) steht, der durch Gruppen $R^1$ und $R^2$ substituiert ist, die in ortho-Stellung zueinander stehen, wobei $R^1$ und $R^2$ unabhängig voneinander für Hydroxy oder einen in vivo hydrolysierbaren Ester davon stehen, wobei der/die Benzol-Ring oder -Ringe gegebenenfalls weiter substituiert sind.

3. Verbindung nach Anspruch 1 oder Anspruch 2, wobei -Y-für eine kovalente Bindung steht.

4. Verbindung nach Anspruch 1 oder Anspruch 2, wobei -Y-für $-N(R^4)CH_2-$ steht.

5. Verbindung nach Anspruch 4, wobei $R^4$ für Wasserstoff steht.

6. Verbindung nach Anspruch 1, bei der es sich um 3-(3,4-Dihydroxybenzylaminomethyl)-7-[2-(2-aminothiazol-4-yl)-2-((Z)-1-carboxy-1-methylethoxyimino)acetamido]ceph-3-em-4-carbonsäure; oder 3-(3,4-Dihydroxybenzyl)-7-[2-(2-aminothiazol-4-yl)-2-((Z)-1-carboxy-1-methylethoxyimino)acetamido]ceph-3-em-4-carbonsäure handelt.

7. Pharmazeutische Zusammensetzung, die eine Verbindung nach einem der Ansprüche 1 bis 6 und einen pharmazeutisch geeigneten Träger enthält.

8. Verfahren zur Herstellung einer Verbindung nach Anspruch 1, bei dem:
(a) eine Cephalosporin-Verbindung mit der Formel:

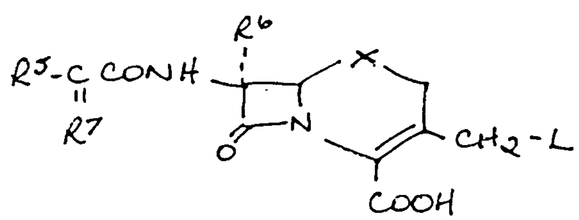

in der $R^5$, $R^6$ und $R^7$ wie oben definiert sind und L für eine Austrittsgruppe steht, mit einer Quelle für -Y-Q, wobei Y und Q wie oben definiert sind, umgesetzt wird;
(b) zur Herstellung einer Verbindung, bei der Y für eine Gruppe -V-Y'- steht, eine Cephalosporin-Verbindung mit der Formel:

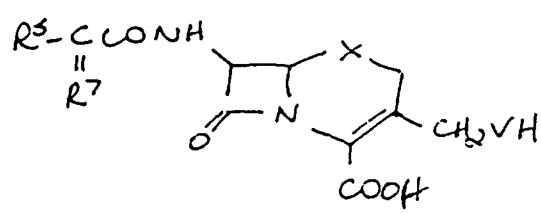

mit einer Quelle für -Y'-Q, wobei $R^5$, $R^6$, $R^7$, V, Y' und Q wie oben definiert sind, umgesetzt wird;
(c) zur Herstellung von Verbindungen mit der in Anspruch 1 definierten Formel (IV) eine Verbindung mit der Formel (VII) mit einer Verbindung mit der Formel (VIII) oder einem reaktiven Derivat davon umgesetzt wird:

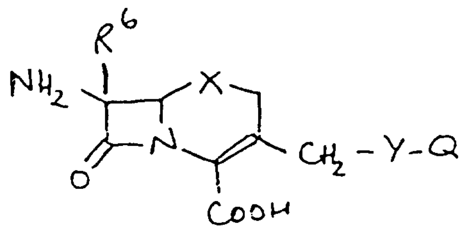

(VII)

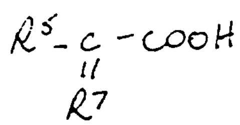

(VIII)

wobei $R^5$, $R^6$, $R^7$ und X wie in Anspruch 1 definiert sind, oder

(d) für Verbindungen mit der Formel (IV), in der $R^7$ für eine Gruppe $=NOR^8$ steht, eine Verbindung mit der Formel (IX):

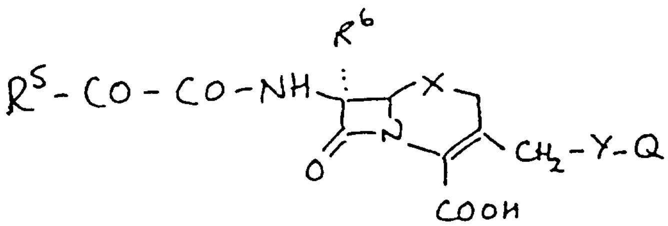

(IX)

in der $R^5$, $R^6$, X, Y und Q wie oben definiert sind, mit einer Verbindung mit der Formel: $R^8ONH_2$, wobei $R^8$ wie in Anspruch 6 definiert ist, umgesetzt wird, oder

(e) für Verbindungen mit der Formel (IV), in der $R^7$ für eine Gruppe $=NOR^8$ steht und $R^8$ für etwas anderes als Wasserstoff steht, eine Verbindung mit der Formel (IV) wie oben definiert, wobei $R^7$ für eine Gruppe $=NOH$ steht, mit einer Verbindung mit der Formel (X):

$L^1 - R^{16}$ (X)

in der $L^1$ für eine Austrittsgruppe steht und $R^{16}$ für eine Gruppe $R^8$ steht, bei der es sich um etwas anderes als Wasserstoff handelt, umgesetzt wird; oder

(f) für Verbindungen mit der Formel (IV) eine Gruppe $R^5$ durch Cyclisierung eines geeigneten Vorläufers davon gebildet wird;

wobei alle funktionellen Gruppen gegebenenfalls geschützt sind;

und wonach, falls erforderlich:

i) jede Schutzgruppe entfernt wird,

ii) zur Herstellung von in vivo hydrolysierbaren Estern korrespondierende Hydroxy-Gruppen verestert werden,

iii) Verbindungen, bei denen X für S steht zu Verbindungen umgewandelt werden, bei denen X für Sulfinyl steht und umgekehrt,

iv) Verbindungen, bei denen $R^4$ für einen Wert steht, in Verbindungen umgewandelt werden, bei denen $R^4$ einen anderen Wert hat,

v) ein pharmazeutisch geeignetes Salz gebildet wird.

**9.** Verbindung mit der Formel (VII) oder (IX) wie in Anspruch 8 definiert, oder eine Verbindung mit der Formel (XI):

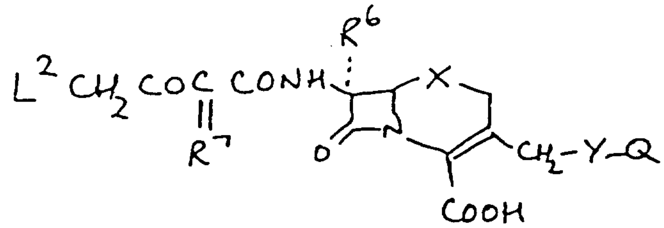 (XI)

in der $L^2$ für eine Austrittsgruppe steht und $R^7$, $R^6$, X, Y und Q wie in Anspruch 8 definiert sind.

**Patentansprüche für folgende Vertragsstaaten : ES, GR**

**1.** Verfahren zur Herstellung einer Cephalosporin-Verbindung mit der folgenden Formel (IV):

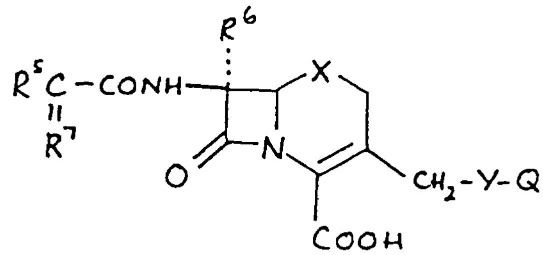 (IV)

oder eines Salzes oder Esters davon; in der: X für Schwefel, Sauerstoff, Methylen oder Sulfinyl steht;
$R^6$ für Wasserstoff, Methoxy oder Formamido steht;
$R^5$ für 2-Aminothiazol-4-yl oder 2-Aminooxazol-4-yl steht, die jeweils gegebenenfalls in der 5-Stellung durch Fluor, Chlor oder Brom substituiert sind, oder in der $R^5$ für 5-Aminoisothiazol-3-yl, 5-Amino-1,2,4-thiadiazol-3-yl, 3-Aminopyrazol-5-yl, 3-Aminopyrazol-4-yl, 2-Aminopyrimidin-5-yl, 2-Aminopyrid-6-yl, 4-Aminopyrimidin-2-yl, 2-Amino-1,3,4-thiadiazol-5-yl oder 5-Amino-1-methyl-1,2,4-triazol-3-yl steht;
$R^7$ für die Formel $=N.O.R^8$ steht (die an der Doppelbindung die syn-Konfiguration aufweist), wobei $R^8$ für folgendes steht: Wasserstoff, (1-6C)Alkyl, (3-8C)Cycloalkyl, (1-3C)Alkyl(3-6C)cycloalkyl, (3-6C)-Cycloalkyl(1-3C)alkyl, (3-6C)Alkenyl, gegebenenfalls durch Carboxy substituiert, (5-8C)Cycloalkenyl, (3-6C)Alkinyl, (2-5C)Alkylcarbamoyl, Phenylcarbamoyl, Benzylcarbamoyl, (1-4C)Alkylcarbamoyl(1-4C)alkyl, Di(1-4C)alkylcarbamoyl(1-4C)alkyl, (1-4C)Halogenalkylcarbamoyl(1-4C)alkyl, (1-3C)Halogenalkyl, (2-6C)-Hydroxyalkyl, (1-4C)Alkoxy(2-4C)alkyl, (1-4C)Alkylthio(2-4C)alkyl, (1-4C)Alkansulfinyl(1-4C)alkyl, (1-4C)-Alkansulfonyl(1-4C)alkyl, (2-6C)Aminoalkyl, (1-4C)Alkylamino(1-6C)alkyl, (2-8C)Dialkylamino(2-6C)alkyl, (1-5C)Cyanoalkyl, 3-Amino-3-carboxypropyl, 2-(Amidinothio)ethyl, 2-(N-Aminoamidinothio)ethyl, Tetrahydropyran-2-yl, Thietan-3-yl, 2-Oxopyrrolidinyl oder 2-Oxotetrahydrofuranyl; oder in der $R^8$ die Formel V hat:-

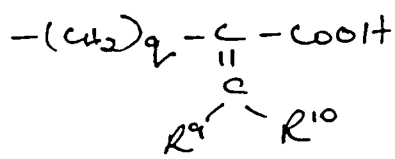 (V)

in der q für 1 oder 2 steht und $R^9$ und $R^{10}$ unabhängig voneinander für Wasserstoff oder $C_{1-4}$-Alkyl stehen; oder in der $R^8$ die Formel VI hat:-

$-CR^{11}R^{12}-(CH_2)_r-COR^{13}$ (VI)

in der r für 0 - 3 steht, $R^{11}$ für Wasserstoff, (1-3C)Alkyl oder Methylthio steht, $R^{12}$ für Wasserstoff, (1-3C)Alkyl, (3-7C)Cycloalkyl, Cyano, Carboxy, (2-5C)Carboxyalkyl oder Methansulfonylamino steht, oder

in der $R^{11}$ und $R^{12}$ zusammen mit dem Kohlenstoff, an den sie gebunden sind, unter Bildung eines (3-7C)carbocyclischen Rings verbunden sind, und in der $R^{13}$ für Hydroxy, Amino, (1-4C)Alkoxy, (1-4C)-Alkylamino oder für die Formel $NHOR^{14}$ steht, wobei $R^{14}$ für Wasserstoff oder (1-4C)Alkyl steht;
oder in der $R^7$ für die Formel $=CH.R^{15}$ stehen kann, in der $R^{15}$ für Wasserstoff, Halogen, (1-6C)Alkyl, (3-7C)Cycloalkyl, (2-6C)Alkenyl, (3-7C)Cycloalkenyl, Phenyl oder Benzyl steht;
Q steht für:

(i) einen Benzol-Ring (der gegebenenfalls an einen weiteren Benzol-Ring unter Bildung einer Naphthyl-Gruppe kondensiert ist oder der gegebenenfalls an eine 5- oder 6-gliedrige heterocyclische aromatische Gruppe, die 1, 2 oder 3 aus Stickstoff, Sauerstoff und Schwefel ausgewählte Heteroatome enthält, kondensiert ist), wobei der Benzol-Ring durch Gruppen $R^1$ und $R^2$, die sich in ortho-Stellung zueinander befinden, substituiert ist, wobei $R^1$ für Hydroxy steht und $R^2$ für Hydroxy steht;
(ii) eine Gruppe mit der Formel (II):

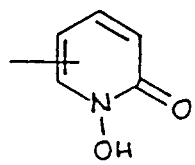

oder;
(iii) eine Gruppe mit der Formel (III):

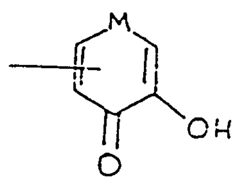

in der M für Sauerstoff oder eine Gruppe $NR^3$ steht, wobei $R^3$ für Wasserstoff oder $C_{1-4}$-Alkyl steht, wobei der Ring Q (oder jeder Benzol-Ring, falls Ring Q für einen Benzol-Ring steht, der an einen anderen Benzol-Ring kondensiert ist) gegebenenfalls mit folgendem weitersubstituiert ist: $C_{1-4}$-Alkyl, Halogen, Hydroxy, Hydroxy-$C_{1-4}$-alkyl, Cyano, Trifluormethyl, Nitro, Amino, $C_{1-4}$-Alkylamino, Di-$C_{1-4}$-alkylamino, Amino-$C_{1-4}$-alkyl, $C_{1-4}$-alkylamino-$C_{1-4}$-alkyl, Di-$C_{1-4}$-alkylamino-$C_{1-4}$-alkyl, $C_{1-4}$-Alkanoyl, $C_{1-4}$-Alkoxy, $C_{1-4}$-Alkylthio, $C_{1-4}$-Alkanoyloxy, Carbamoyl, $C_{1-4}$-Alkylcarbamoyl, Di-$C_{1-4}$-alkylcarbamoyl, Carboxy, Carboxy-$C_{1-4}$-alkyl, Sulfo, Sulfo-$C_{1-4}$-alkyl, $C_{1-4}$-Alkansulfonamido, $C_{1-4}$-Alkoxycarbonyl, $C_{1-4}$-Alkanoylamino, Nitroso, Thioureido, Amidino, Ammonium, Mono-, Di- oder Tri-$C_{1-4}$-alkylammonium oder Pyridinium, oder einem 5-gliedrigen 1 bis 4 aus Sauerstoff, Stickstoff und Schwefel ausgewählte Heteroatome enthaltenden heterocyclischen Ring, der gegebenenfalls mit 1, 2 oder 3 $C_{1-4}$-Alkyl- oder $C_{1-4}$-Alkoxy-Gruppen substituiert ist;
Y, das an den Benzol-Ring oder den Ring mit der Formel (II) oder (III) bindet, für eine kovalente Bindung oder für eine Gruppe -V-Y'- steht, wobei V für Sauerstoff, Schwefel oder eine Gruppe $-NR^4-$ steht und -Y'-für eine kovalente Bindung oder $C_{1-4}$-Alkenylen steht, wobei $R^4$ für Wasserstoff, $C_{1-4}$-Alkyl, das gegebenenfalls mit Halogen, Hydroxy, $C_{1-4}$-Alkoxy, Carboxy, Amino, Cyano, $C_{1-4}$-Alkanoylamino oder Phenyl substituiert ist, steht, oder wobei $R^4$ für $C_{2-6}$-Alkenyl, $C_{1-4}$-Alkansulfonyl oder $C_{1-4}$-Alkanoyl steht;
wobei bei dem Verfahren:

(a) eine Cephalosporin-Verbindung mit der Formel:

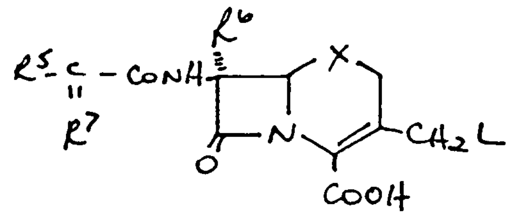

in der $R^5$, $R^6$ und $R^7$ wie oben definiert sind und L für eine Austrittsgruppe steht, mit einer Quelle für -Y-Q, wobei Y und Q wie oben definiert sind, umgesetzt wird;
(b) zur Herstellung einer Verbindung, bei der Y für eine Gruppe -V-Y'- steht, eine Cephalosporin-Verbindung mit der Formel:

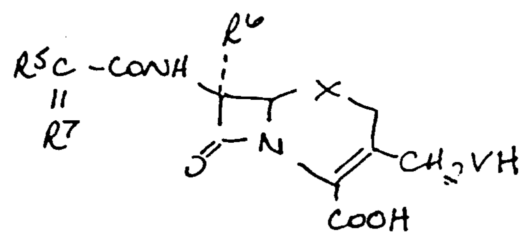

mit einer Quelle für -Y'-Q, wobei $R^5$, $R^6$, $R^7$, V, Y' und Q wie oben definiert sind, umgesetzt wird;
wobei alle funktionellen Gruppen gegebenenfalls geschützt sind, und wonach, falls erforderlich:
i) jede Schutzgruppe entfernt wird,
ii) zur Herstellung von in vivo hydrolysierbaren Estern korrespondierende Hydroxy-Gruppen verestert werden,
iii) Verbindungen, bei denen $R^4$ für einen Wert steht, in Verbindungen umgewandelt werden, bei denen $R^4$ einen anderen Wert hat,
iv) ein pharmazeutisch geeignetes Salz gebildet wird.

**2.** Verfahren zur Herstellung einer Verbindung mit der Formel (IV), in der $R^5$, $R^6$, $R^7$, Y und Q wie in Anspruch 1 definiert sind:

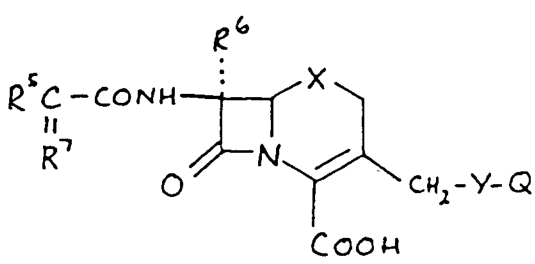

(IV)

wobei bei dem Verfahren:
i) eine Verbindung mit der Formel (VII) mit einer Verbindung mit der Formel (VIII) oder einem reaktiven Derivat davon umgesetzt wird:

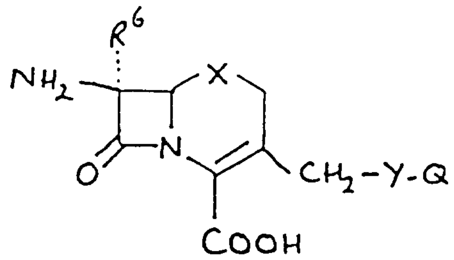

(VII)

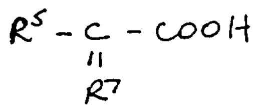

(VIII)

wobei $R^5$, $R^6$, $R^7$ und X wie oben definiert sind; oder

(ii) für Verbindungen mit der Formel (IV), in der $R^7$ für eine Gruppe $=NOR^8$ steht, eine Verbindung mit der Formel (IX):

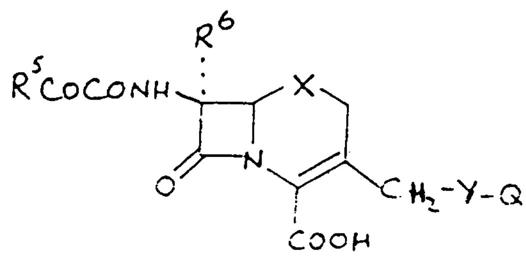

(IX)

in der $R^5$, $R^6$, X, Y und Q wie oben definiert sind, mit einer Verbindung mit der Formel: $R^8ONH_2$, wobei $R^8$ wie oben definiert ist, umgesetzt wird; oder

(iii) für Verbindungen mit der Formel (IV), in der $R^7$ für eine Gruppe $=NOR^8$ steht und $R^8$ für etwas anderes als Wasserstoff steht, eine Verbindung mit der Formel (IV) wie oben definiert, wobei $R^7$ für eine Gruppe $=NOH$ steht, mit einer Verbindung mit der Formel (X):

$L^1 - R^{16}$ (X)

in der $L^1$ für eine Austrittsgruppe steht und $R^{16}$ für eine Gruppe $R^8$ steht, bei der es sich um etwas anderes als Wasserstoff handelt, umgesetzt wird; oder

(iv) für Verbindungen mit der Formel (IV) eine Gruppe $R^5$ durch Cyclisierung eines geeigneten Vorläufers davon gebildet wird;

wobei alle funktionellen Gruppen gegebenenfalls geschützt sind;

und wonach, falls erforderlich:

i) jede Schutzgruppe entfernt wird,

ii) zur Herstellung von in vivo hydrolysierbaren Estern korrespondierende Hydroxy-Gruppen verestert werden,

iii) Verbindungen, bei denen X für S steht zu Verbindungen umgewandelt werden, bei denen X für Sulfinyl steht und umgekehrt,

iv) Verbindungen, bei denen $R^4$ für einen Wert steht, in Verbindungen umgewandelt werden, bei denen $R^4$ einen anderen Wert hat,

v) ein pharmazeutisch geeignetes Salz gebildet wird.

**3.** Verfahren nach Anspruch 1 oder Anspruch 2, bei dem eine Verbindung hergestellt wird, bei der Q für einen Benzol-Ring (der gegebenenfalls unter Bildung einer Naphthyl-Gruppe an einen weiteren Benzol-Ring kondensiert ist) steht, der durch Gruppen $R^1$ und $R^2$ substituiert ist, die in ortho-Stellung zueinander stehen, wobei $R^1$ und $R^2$ für Hydroxy stehen, wobei der/die Benzol-Ring oder -Ringe gegebenenfalls weiter substituiert sind.

**4.** Verfahren nach einem der Ansprüche 1 bis 3 zur Herstellung einer Verbindung, bei der -Y- für eine kovalente Bindung steht.

**5.** Verfahren nach einem der Ansprüche 1 bis 3 zur Herstellung einer Verbindung, bei der -Y- für $-N(R^4)-CH_2$-steht.

**6.** Verfahren nach Anspruch 5 zur Herstellung einer Verbindung, bei der $R^4$ für Wasserstoff steht.

**7.** Verfahren zur Herstellung einer pharmazeutischen Zusammensetzung, bei dem eine Cephalosporin-Verbindung mit der Formel (IV) wie in Anspruch 1 definiert und ein pharmazeutisch geeigneter Träger zusammengebracht werden.

**Revendications**
**Revendications pour les Etats contractants suivants : AT, BE, CH, DE, FR, GB, IT, LI, LU, NL, SE**

**1.** Céphalosporine de formule (IV) :

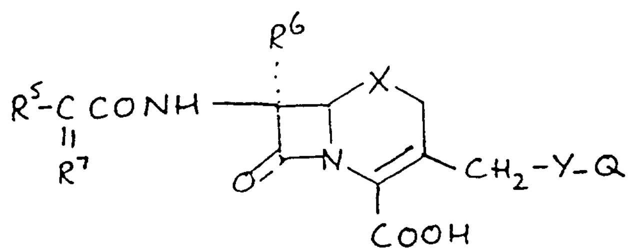

(IV)

ou un de ses sels ou esters ; formule dans laquelle :

X représente le soufre, l'oxygène, un groupe méthylène ou sulfinyle ;

$R^6$ représente l'hydrogène, un groupe méthoxy ou formamido ;

$R^5$ représente un groupe 2-aminothiazole-4-yle ou 2-aminooxazole-4-yle, chacun facultativement substitué en position 5 avec le fluor, le chlore ou le brome, ou bien $R^5$ représente un groupe 5-aminoisothiazole-3-yle, 5-amino-1,2,4-thiadiazole-3-yle, 3-aminopyrazole-5-yle, 3-aminopyrazole-4-yle, 2-aminopyrimidine-5-yle, 2-aminopyrid-6-yle, 4-aminopyrimidine-2-yle, 2-amino-1,3,4-thiadiazole-5-yle ou 5-amino-1-méthyl-1,2,4-triazole-3-yle ;

$R^7$ répond à la formule $=N.O.R^8$ (possédant la configuration syn de part et d'autre de la double liaison) dans laquelle $R^8$ représente l'hydrogène, un groupe alkyle en $C_1$ à $C_6$, cycloalkyle en $C_3$ à $C_8$, (alkyle en $C_1$ à $C_3$)-(cycloalkyle en $C_3$ à $C_6$), (cycloalkyle en $C_3$ à $C_6$)-(alkyle en $C_1$ à $C_3$), alcényle en $C_3$ à $C_6$, facultativement substitué avec un groupe carboxy, cycloalcényle en $C_5$ à $C_8$, alcynyle en $C_3$ à $C_6$, (alkyle en $C_2$ à $C_5$)-carbamoyle, phénylcarbamoyle, benzylcarbamoyle, (alkyle en $C_1$ à $C_4$)-carbamoyl-(alkyle en $C_1$ à $C_4$), di-(alkyle en $C_1$ à $C_4$)-carbamoyl-(alkyle en $C_1$ à $C_4$), (halogénalkyle en $C_1$ à $C_4$)-carbamoyl-(alkyle en $C_1$ à $C_4$), halogénalkyle en $C_1$ à $C_3$, hydroxyalkyle en $C_2$ à $C_6$, (alkoxy en $C_1$ à $C_4$)-(alkyle en $C_2$ à $C_4$), (alkyle en $C_1$ à $C_4$)-thio-(alkyle en $C_2$ à $C_4$), (alcane en $C_1$ à $C_4$)-sulfinyl-(alkyle en $C_1$ à $C_4$), (alcane en $C_1$ à $C_4$)-sulfonyl-(alkyle en $C_1$ à $C_4$), aminoalkyle en $C_2$ à $C_6$, (alkyle en $C_1$ à $C_4$)-amino-(alkyle en $C_1$ à $C_6$), (dialkyle en $C_2$ à $C_8$)-amino-(alkyle en $C_2$ à $C_6$, cyanalkyle en $C_1$ à $C_5$, 3-amino-3-carboxypropyle, 2-(amidinothio)éthyle, 2-(N-aminoamidinothio)éthyle, tétrahydropyranne-2-yle, thiétanne-3-yle, 2-oxopyrrolidinyle ou 2-oxotétrahydrofurannyle, ou $R^8$ répond à la formule V :

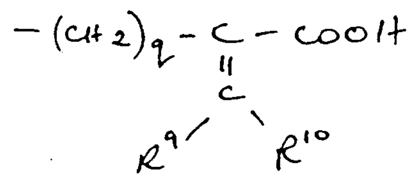

(V)

dans laquelle Q est égal à un ou deux et $R^9$ et $R^{10}$ représentent indépendamment l'hydrogène ou un groupe alkyle en $C_1$ à $C_4$ ; ou bien $R^8$ répond à la formule VI :

$-CR^{11}R^{12}-(CH_2)_r-COR^{13}$ (VI)

dans laquelle r a une valeur de 0 à 3, $R^{11}$ représente l'hydrogène, un groupe alkyle en $C_1$ à $C_3$ ou méthylthio, $R^{12}$ représente l'hydrogène, un groupe alkyle en $C_1$ à $C_3$, cycloalkyle en $C_3$ à $C_7$, cyano, carboxy, carboxyalkyle en $C_2$ à $C_5$ ou méthanesulfonylamino, ou bien $R^{11}$ et $R^{12}$ sont réunis en formant, conjointement avec l'atome de carbone auquel ils sont fixés, un noyau carbocyclique en $C_3$ à $C_7$, et $R^{13}$ représente un groupe hydroxy, amino, alkoxy en $C_1$ à $C_4$, alkylamino en $C_1$ à $C_4$ ou un groupe de formule $NHOR^{14}$ dans laquelle $R^{14}$ représente l'hydrogène ou un groupe alkyle en $C_1$ à $C_4$ ;

ou bien $R^7$ peut répondre à la formule $=CH.R^{15}$ dans laquelle $R^{15}$ représente l'hydrogène, un halogène, un groupe alkyle en $C_1$ à $C_6$, cycloalkyle en $C_3$ à $C_7$, alcényle en $C_2$ à $C_6$, cycloalcényle en $C_3$ à $C_7$, phényle ou benzyle ;

Q représente :

(i) un noyau benzénique (facultativement condensé avec un autre noyau benzénique en formant ainsi un groupe naphtyle, ou bien facultativement condensé avec un groupe aromatique hétérocyclique penta- ou hexagonal contenant 1, 2 ou 3 hétéroatomes choisis entre l'azote, l'oxygène et le soufre), ledit noyau benzénique étant substitué avec les groupes $R^1$ et $R^2$ qui sont en position ortho l'un par rapport à l'autre, $R^1$ représente un groupe hydroxy et $R^2$ représente un groupe hydroxy ;

(ii) un groupe de formule (II) :

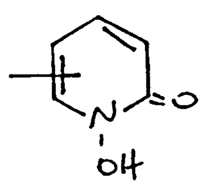

ou

(iii) un groupe de formule (III) :

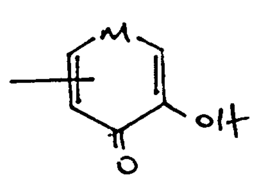

dans laquelle M représente l'oxygène ou un groupe $NR^3$, dans lequel $R^3$ représente l'hydrogène ou un groupe alkyle en $C_1$ à $C_4$,

ledit noyau Q (ou, dans le cas où le noyau Q est un noyau benzénique condensé avec un autre noyau benzénique, chaque noyau benzénique) étant facultativement substitué en outre avec un groupe alkyle en $C_1$ à $C_4$, halogéno, hydroxy, hydroxyalkyle en $C_1$ à $C_4$, cyano, trifluorométhyle, nitro, amino, alkylamino en $C_1$ à $C_4$, di-(alkyle en $C_1$ à $C_4$)-amino, amino-(alkyle en $C_1$ à $C_4$), (alkyle en $C_1$ à $C_4$)-amino-(alkyle en $C_1$ à $C_4$), di-(alkyle en $C_1$ à $C_4$)-amino-(alkyle en $C_1$ à $C_4$), alcanoyle en $C_1$ à $C_4$, alkoxy en $C_1$ à $C_4$, alkylthio en $C_1$ à $C_4$, alcanoyloxy en $C_1$ à $C_4$, carbamoyle, (alkyle en $C_1$ à $C_4$)-carbamoyle, di-(alkyle en $C_1$ à $C_4$)-carbamoyle, carboxy, carboxy-(alkyle en $C_1$ à $C_4$), sulfo, sulfoalkyle en $C_1$ à $C_4$, alcanesulfamido en $C_1$ à $C_4$, (alkoxy en $C_1$ à $C_4$)-carbonyle, alcanoylamino en $C_1$ à $C_4$, nitroso, thiouréido, amidino, ammonium, mono-, di- ou tri(alkyle en $C_1$ à

$C_4$)-ammonium ou -pyridinium, ou un noyau hétérocyclique pentagonal contenant 1 à 4 hétéroatomes choisis entre l'oxygène, l'azote et le soufre, qui est facultativement substitué avec 1, 2 ou 3 groupes alkyle en $C_1$ à $C_4$ ou alkoxy en $C_1$ à $C_4$ ;

Y, qui assure la liaison avec le noyau benzénique ou le noyau de formule (II) ou (III), est une liaison covalente ou représente un groupe -V-Y'- dans lequel V représente l'oxygène, le soufre ou un groupe -$NR^4$- et -Y'-représente une liaison covalente ou un groupe alkylène en $C_1$ à $C_4$, $R^4$ représentant l'hydrogène, un groupe alkyle en $C_1$ à $C_4$, facultativement substitué avec un groupe quelconque choisi entre des groupes halogène, hydroxy, alkoxy en $C_1$ à $C_4$, carboxy, amino, cyano, alcanoylamino en $C_1$ à $C_4$ ou phényle, ou bien $R^4$ représentant un groupe alcényle en $C_2$ à $C_6$, alcanesulfonyle en $C_1$ à $C_4$ ou alcanoyle en $C_1$ à $C_4$.

**2.** Composé suivant la revendication 1, dans lequel Q représente un noyau benzénique (facultativement condensé avec un autre noyau benzénique en formant ainsi un groupe napthyle), substitué avec des groupes $R^1$ et $R^2$ qui sont en positions ortho l'un par rapport à l'autre, $R^1$ et $R^2$ représentant indépendamment un groupe hydroxy ou un de ses esters hydrolysables in vivo, ledit ou lesdits noyaux benzéniques étant en outre facultativement substitués.

**3.** Composé suivant la revendication 1 ou la revendication 2, dans lequel -Y- représente une liaison covalente.

**4.** Composé suivant la revendication 1 ou la revendication 2, dans lequel -Y- représente un groupe -$N(R^4)$-$CH_2$-.

**5.** Composé suivant la revendication 4, dans lequel $R^4$ représente l'hydrogène.

**6.** Composé suivant la revendication 1, qui est l'acide 3-(3,4-dihydroxybenzylaminométhyl)-7-[2-(2-aminothiazole-4-yl)-2-((Z)-1-carboxy-1-méthyléthoxyimino)acétamido]céph-3-ème-4-carboxylique ; ou l'acide 3-(3,4-dihydroxybenzyl)-7-[2-(2-aminothiazole-4-yle)-2-((Z)-1-carboxy-1-méthyléthoxyimino)acétamido]-céph-3-ème-4-carboxylique.

**7.** Composition pharmaceutique qui comprend un composé suivant l'une quelconque des revendications 1 à 6 et un support pharmaceutiquement acceptable.

**8.** Procédé de préparation d'un composé suivant la revendication 1, procédé qui comprend :

(a) la réaction d'une céphalosporine de formule :

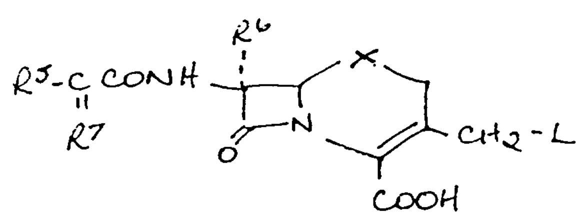

dans laquelle $R^5$, $R^6$ et $R^7$ répondent aux définitions précitées et L représente un groupe partant, avec une source de groupe -Y-Q- dans lequel Y et Q répondent aux définitions précitées ;

(b) pour la préparation d'un composé dans lequel Y représente un groupe -V-Y'-, la réaction d'une céphalosporine de formule :

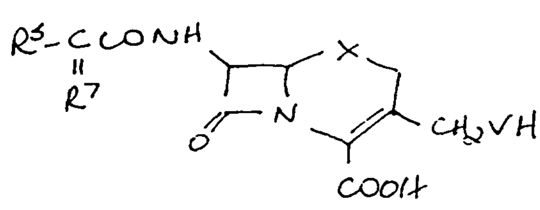

avec une source d'un groupe -Y'-Q, formules dans lesquelles $R^5$, $R^6$, $R^7$, V, Y' et Q répondent aux définitions précitées ;

(c) pour la préparation de composés répondant à la formule (IV) définie dans la revendication 1, la réaction d'un composé de formule (VII) avec un composé de formule (VIII) ou un de ses dérivés réactifs :

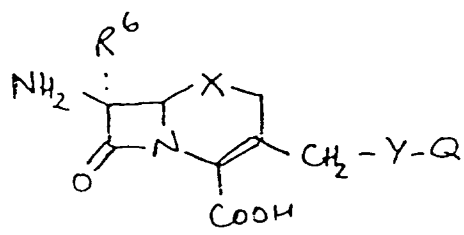

(VII)

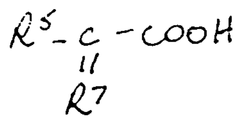

(VIII)

formules dans lesquelles $R^5$, $R^6$, $R^7$ et X sont tels que définis dans la revendication 1 ; ou

d) pour des composés de formule (IV) dans laquelle $R^7$ représente un groupe $=NOR^8$, la réaction d'un composé de formule (IX) :

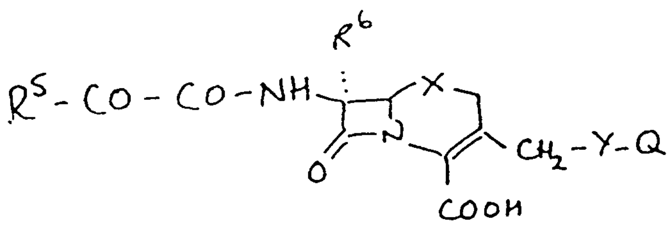

(IX)

dans laquelle $R^5$, $R^6$, X, Y et Q répondent aux définitions précitées, avec un composé de fromule : $R^8ONH_2$ dans laquelle $R^8$ est tel que défini dans la revendication 6 ; ou

e) pour des composés de formule (IV) dans laquelle $R^7$ représente un groupe $=NOR^8$ et $R^8$ est autre que l'hydrogène, la réaction d'un composé de formule (IV) répondant à la définition précitée dans laquelle $R^7$ représente un groupe $=NOH$ avec un composé de formule (X) :

$L^1 - R^{16}$ (X)

dans laquelle $L^1$ représente un groupe partant et $R^{16}$ représente un groupe $R^8$ autre que l'hydrogène ; ou

f) pour des composés de formule (IV), la formation d'un groupe $R^5$ par cyclisation d'un de leurs précurseurs appropriés,

dans lequel n'importe quels groupes fonctionnels sont facultativement protégés ;

puis, si nécessaire :

i) l'élimination de n'importe quel groupe protecteur,

ii) pour la préparation d'esters hydrolysables in vivo, l'estérification des groupes hydroxy correspondants,

iii) la transformation de composés dans lesquels X représente S en composés dans lesquels X représente un groupe sulfinyle, et vice versa,

iv) la transformation de composés dans lesquels $R^4$ répond à une définition en composés dans lesquels $R^4$ répond à une autre définition,
v) la formation d'un sel pharmaceutiquement acceptable.

**9.** Composé de formule (VII) ou (IX) tel que défini dans la revendication 8, ou composé de formule (XI) :

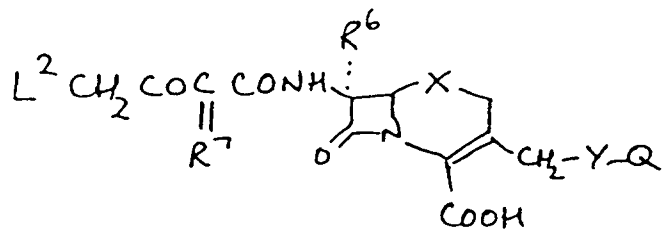

(XI)

dans laquelle $L^2$ représente un groupe partant et $R^7$, $R^6$, X, Y et Q sont tels que définis dans la revendication 8.

**Revendications pour les Etats contractants suivants : ES, GR**

**1.** Procédé de préparation d'une céphalosporine de formule (IV) :

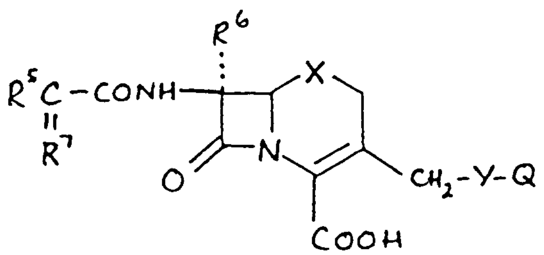

(IV)

ou d'un de ses sels ou esters ; formule dans laquelle :

X représente le soufre, l'oxygène, un groupe méthylène ou sulfinyle ;
$R^6$ représente l'hydrogène, un groupe méthoxy ou formamido ;

$R^5$ représente un groupe 2-aminothiazole-4-yle ou 2-aminooxazole-4-yle, chacun facultativement substitué en position 5 avec le fluor, le chlore ou le brome, ou bien $R^5$ représente un groupe 5-aminoisothiazole-3-yle, 5-amino-1,2,4-thiadiazole-3-yle, 3-aminopyrazole-5-yle, 3-aminopyrazole-4-yle, 2-aminopyrimidine-5-yle, 2-aminopyrid-6-yle, 4-aminopyrimidine-2-yle, 2-amino-1,3,4-thiadiazole-5-yle ou 5-amino-1-méthyl-1,2,4-triazole-3-yle ;

$R^7$ répond à la formule $=N.O.R^8$ (possédant la configuration syn de part et d'autre de la double liaison) dans laquelle $R^8$ représente l'hydrogène, un groupe alkyle en $C_1$ à $C_6$, cycloalkyle en $C_3$ à $C_8$, (alkyle en $C_1$ à $C_3$)-(cycloalkyle en $C_3$ à $C_6$), (cycloalkyle en $C_3$ à $C_6$)-(alkyle en $C_1$ à $C_3$), alcényle en $C_3$ à $C_6$, facultativement substitué avec un groupe carboxy, cycloalcényle en $C_5$ à $C_8$, alcynyle en $C_3$ à $C_6$, (alkyle en $C_2$ à $C_5$)-carbamoyle, phénylcarbamoyle, benzylcarbamoyle, (alkyle en $C_1$ à $C_4$)-carbamoyl-(alkyle en $C_1$ à $C_4$), di-(alkyle en $C_1$ à $C_4$)-carbamoyl-(alkyle en $C_1$ à $C_4$), (halogénalkyle en $C_1$ à $C_4$)-carbamoyl-(alkyle en $C_1$ à $C_4$), halogénalkyle en $C_1$ à $C_3$, hydroxyalkyle en $C_2$ à $C_6$, (alkoxy en $C_1$ à $C_4$)-(alkyle en $C_2$ à $C_4$), (alkyle en $C_1$ à $C_4$)-thio-(alkyle en $C_2$ à $C_4$), (alcane en $C_1$ à $C_4$)-sulfinyl-(alkyle en $C_1$ à $C_4$), (alcane en $C_1$ à $C_4$)-sulfonyl-(alkyle en $C_1$ à $C_4$), aminoalkyle en $C_2$ à $C_6$, (alkyle en $C_1$ à $C_4$)-amino-(alkyle en $C_1$ à $C_6$), (dialkyle en $C_2$ à $C_8$)-amino-(alkyle en $C_2$ à $C_6$, cyanalkyle en $C_1$ à $C_5$, 3-amino-3-carboxypropyle, 2-(amidinothio)éthyle, 2-(N-aminoamidinothio)éthyle, tétrahydropyranne-2-yle, thiétanne-3-yle, 2-oxopyrrolidinyle ou 2-oxotétrahydrofurannyle, ou $R^8$ répond à la formule V :

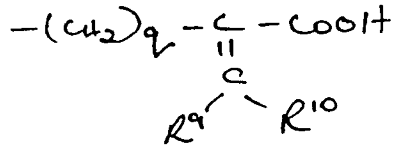

(V)

dans laquelle Q est égal à un ou deux et $R^9$ et $R^{10}$ représentent indépendamment l'hydrogène ou un groupe alkyle en $C_1$ à $C_4$ ; ou bien $R^8$ répond à la formule VI :

$-CR^{11}R^{12}-(CH_2)_r-COR^{13}$ (VI)

dans laquelle r a une valeur de 0 à 3, $R^{11}$ représente l'hydrogène, un groupe alkyle en $C_1$ à $C_3$ ou méthylthio, $R^{12}$ représente l'hydrogène, un groupe alkyle en $C_1$ à $C_3$, cycloalkyle en $C_3$ à $C_7$, cyano, carboxy, carboxyalkyle en $C_2$ à $C_5$ ou méthanesulfonylamino, ou bien $R^{11}$ et $R^{12}$ sont réunis en formant, conjointement avec l'atome de carbone auquel ils sont fixés, un noyau carbocyclique en $C_3$ à $C_7$, et $R^{13}$ représente un groupe hydroxy, amino, alkoxy en $C_1$ à $C_4$, alkylamino en $C_1$ à $C_4$ ou un groupe de formule $NHOR^{14}$ dans laquelle $R^{14}$ représente l'hydrogène ou un groupe alkyle en $C_1$ à $C_4$ ;

ou bien $R^7$ peut répondre à la formule $=CH.R^{15}$ dans laquelle $R^{15}$ représente l'hydrogène, un halogène, un groupe alkyle en $C_1$ à $C_6$, cycloalkyle en $C_3$ à $C_7$, alcényle en $C_2$ à $C_6$, cycloalcényle en $C_3$ à $C_7$, phényle ou benzyle ;

Q représente :

(i) un noyau benzénique (facultativement condensé avec un autre noyau benzénique en formant ainsi un groupe naphtyle, ou bien facultativement condensé avec un groupe aromatique hétérocyclique penta- ou hexagonal contenant 1, 2 ou 3 hétéroatomes choisis entre l'azote, l'oxygène et le soufre), ledit noyau benzénique étant substitué avec les groupes $R^1$ et $R^2$ qui sont en position ortho l'un par rapport à l'autre, $R^1$ représente un groupe hydroxy et $R^2$ représente un groupe hydroxy ;

(ii) un groupe de formule (II) :

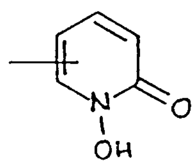

ou

(iii) un groupe de formule (III) :

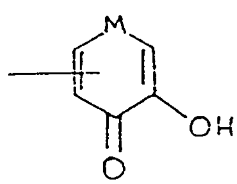

dans laquelle M représente l'oxygène ou un groupe $NR^3$, dans lequel $R^3$ représente l'hydrogène ou un groupe alkyle en $C_1$ à $C_4$,

ledit noyau Q (ou, dans le cas où le noyau Q est un noyau benzénique condensé avec un autre noyau benzénique, chaque noyau benzénique) étant facultativement substitué en outre avec un groupe alkyle en $C_1$ à $C_4$, halogéno, hydroxy, hydroxyalkyle en $C_1$ à $C_4$, cyano, trifluorométhyle, nitro, amino, alkylamino en $C_1$ à $C_4$, di-(alkyle en $C_1$ à $C_4$)-amino, amino-(alkyle en $C_1$ à $C_4$), (alkyle en $C_1$ à $C_4$)-amino-(alkyle en $C_1$ à $C_4$), di-(alkyle en $C_1$ à $C_4$)-amino-(alkyle en $C_1$ à $C_4$), alcanoyle en $C_1$ à $C_4$, alkoxy en $C_1$ à $C_4$, alkylthio en $C_1$ à $C_4$, alcanoyloxy en $C_1$ à $C_4$, carbamoyle, (alkyle en $C_1$ à $C_4$)-carbamoyle, di-(alkyle en $C_1$ à $C_4$)-carbamoyle, carboxy, carboxy-(alkyle en $C_1$ à $C_4$), sulfo, sulfoalkyle en $C_1$ à $C_4$, alcanesulfamido en $C_1$ à $C_4$, (alkoxy en $C_1$ à $C_4$)-carbonyle, alcanoylamino en $C_1$ à $C_4$, nitroso, thiouréido, amidino, ammonium, mono-, di- ou tri(alkyle en $C_1$ à

$C_4$)-ammonium ou -pyridinium, ou un noyau hétérocyclique pentagonal contenant 1 à 4 hétéroatomes choisis entre l'oxygène, l'azote et le soufre, qui est facultativement substitué avec 1, 2 ou 3 groupes alkyle en $C_1$ à $C_4$ ou alkoxy en $C_1$ à $C_4$ ;

Y, qui assure la liaison avec le noyau benzénique ou le noyau de formule (II) ou (III), est une liaison covalente ou représente un groupe -V-Y'- dans lequel V représente l'oxygène, le soufre ou un groupe $-NR^4-$ et -Y'-représente une liaison covalente ou un groupe alkylène en $C_1$ à $C_4$, $R^4$ représentant l'hydrogène, un groupe alkyle en $C_1$ à $C_4$, facultativement substitué avec un groupe quelconque choisi entre des groupes halogène, hydroxy, alkoxy en $C_1$ à $C_4$, carboxy, amino, cyano, alcanoylamino en $C_1$ à $C_4$ ou phényle, ou bien $R^4$ représentant un groupe alcényle en $C_2$ à $C_6$, alcanesulfonyle en $C_1$ à $C_4$ ou alcanoyle en $C_1$ à $C_4$, procédé qui comprend :

(a) la réaction d'une céphalosporine de formule :

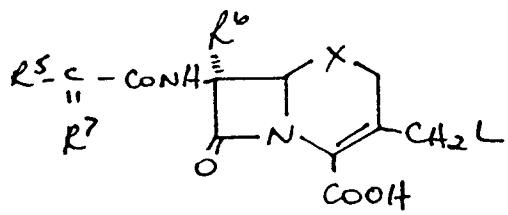

dans laquelle $R^5$, $R^6$ et $R^7$ répondent aux définitions précitées et L représente un groupe partant, avec une source de groupe -Y-Q dans lequel Y et Q répondent aux définitions précitées ;

(b) pour la préparation d'un composé dans lequel Y représente un groupe -V-Y'-, la réaction d'une céphalosporine de formule :

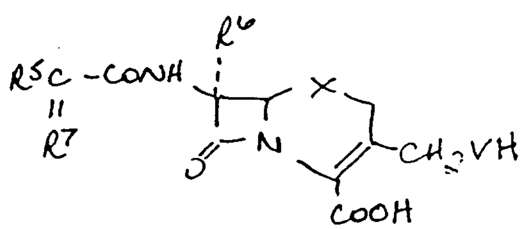

avec une source d'un groupe -Y'-Q, formules dans lesquelles $R^5$, $R^6$, $R^7$, V, Y' et Q répondent aux définitions précitées ;

n'importe quels groupes fonctionnels étant facultativement protégés ;

puis, si nécessaire :

i) l'élimination de n'importe quel groupe protecteur,

ii) pour la préparation d'esters hydrolysables in vivo, l'estérification des groupes hydroxy correspondants,

iii) la transformation de composés dans lesquels $R^4$ répond à une définition en composés dans lesquels $R^4$ répond à une autre définition,

iv) la formation d'un sel pharmaceutiquement acceptable.

**2.** Procédé de préparation d'un composé de formule (IV), dans laquelle $R^5$, $R^6$, $R^7$, Y et Q répondent aux définitions suivant la revendication 1 :

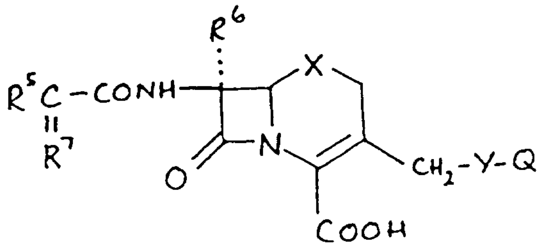

(IV)

procédé qui comprend :

i) la réaction d'un composé de formule (VII) avec un composé de formule (VIII) ou un de ses dérivés réactifs

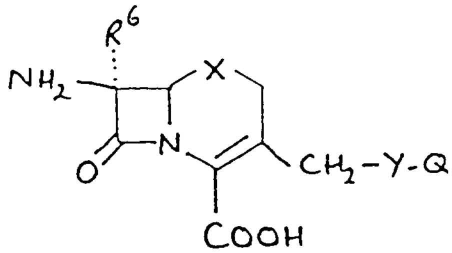

(VII)

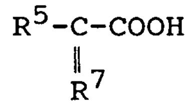

(VIII)

dans lesquelles $R^5$, $R^6$, $R^7$ et X répondent aux définitions précitées ou ii) pour des composés de formule (IV) dans laquelle $R^7$ représente un groupe $=NOR^8$, la réaction d'un composé de formule (IX) :

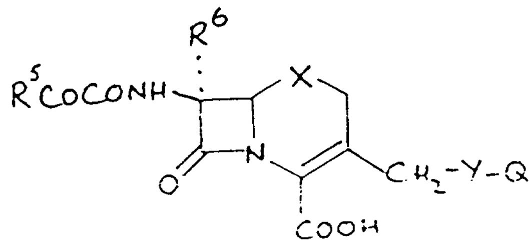

(IX)

dans laquelle $R^5$, $R^6$, X, Y et Q répondent aux définitions précitées, avec un composé de formule : $R^8ONH_2$ dans laquelle $R^8$ répond à la définition précitée ; ou iii) pour des composés de formule (IV) dans laquelle $R^7$ représente un groupe $=NOR^8$ et $R^8$ est autre que l'hydrogène, la réaction d'un composé de formule (IV) répondant à la définition précitée, dans laquelle $R^7$ représente un groupe $=NOH$, avec un composé de formule (X) :

$L^1$ - $R^{16}$ (X)

dans laquelle $L^1$ représente un groupe partant et $R^{16}$ représente un groupe $R^8$ autre que l'hydrogène ; ou iv) pour des composés de formule (IV), la formation d'un groupe $R^5$ par cyclisation d'un de leurs précurseurs appropriés ;

dans lequel n'importe quels groupes fonctionnels sont facultativement protégés ;

puis, si nécessaire :

i) l'élimination de n'importe quel groupe protecteur,

ii) pour la préparation d'esters hydrolysables in vivo, l'estérification des groupes hydroxy correspondants,

iii) la transformation de composés dans lesquels X représente S en composés dans lesquels X représente un groupe sulfinyle, et vice versa,

iv) la transformation de composés dans lesquels $R^4$ répond à une définition en composés dans lesquels $R^4$ répond à une autre définition,

v) la formation d'un sel pharmaceutiquement acceptable.

**3.** Procédé suivant la revendication 1 ou la revendication 2, qui comprend la préparation d'un composé dans lequel Q représente un noyau benzénique (facultativement condensé avec un autre noyau benzénique en formant ainsi un groupe naphtyle) substitué avec des groupes $R^1$ et $R^2$ qui sont en positions ortho l'un par rapport à l'autre, $R^1$ et $R^2$ représentant des groupes hydroxy, ledit ou lesdits noyaux benzéniques étant en outre facultativement substitués.

**4.** Procédé suivant l'une quelconque des revendications 1 à 3 pour la préparation d'un composé dans lequel -Y- représente une liaison covalente.

**5.** Procédé suivant l'une quelconque des revendications 1 à 3 pour la préparation d'un composé dans lequel -Y- représnte un groupe $-N(R^4)CH_2-$ ;

**6.** Procédé suivant la revendication 5 pour la préparation d'un composé dans lequel $R^4$ représente l'hydrogène.

**7.** Procédé de préparation d'une composition pharmaceutique, qui comprend la mise en association d'une céphalosporine de formule (IV) telle que définie dans la revendication 1 et d'un support pharmaceutiquement acceptable.